# EUROPEAN PATENT APPLICATION

(11) **EP 4 623 906 A1**
(43) Date of publication of application: **01.10.2025**
(21) Application number: 23893994.6
(22) Date of filing: 24.11.2023
(51) Int. Cl.: A61K 31/136, A61K 9/08, A61P 27/02

(54) **OPHTHALMIC FORMULATION COMPRISING PYRIDINE PHENYL COMPOUND, PREPARATION METHOD THEREFOR, AND USE THEREOF**

(30) Priority: 26.11.2022 CN 202211479343
(71) Applicant: Zhuhai United Laboratories Co., Ltd., Zhuhai, Guangdong 519040 (CN)
(72) Inventor: YU, Tingting, Zhongshan, Guangdong 528467 (CN); SHEN, Meiyue, Zhongshan, Guangdong 528467 (CN); MU, Liwei, Zhongshan, Guangdong 528467 (CN); SU, Yuan, Zhongshan, Guangdong 528467 (CN); HE, Yuanzhi, Zhongshan, Guangdong 528467 (CN); WANG, Degang, Zhongshan, Guangdong 528467 (CN); WU, Shouting, Zhongshan, Guangdong 528467 (CN)
(74) Representative: Kröncke, Rolf
(86) International application number: PCT/CN2023/133814
(87) International publication number: WO 2024/109905

(57) **Abstract**

The present invention belongs to the field of pharmaceutical preparations and discloses an ophthalmic formulation containing pyridine phenyl compounds, its preparation method, and application. The ophthalmic formulation includes an active ingredient pyridine phenyl compounds and excipient. The active ingredient pyridine phenyl compounds include compounds of formula (II), their isomers, or their pharmaceutically acceptable salts. It can be used for dry eye disease, allergic conjunctivitis, macular degeneration, cataracts, keratoconus, bullous keratopathy, Fuchs endothelial corneal dystrophy, ocular cicatricial pemphigoid, meibomian gland dysfunction, uveitis, scleritis, Stevens-Johnson syndrome, ocular rosacea, Sjögren syndrome.

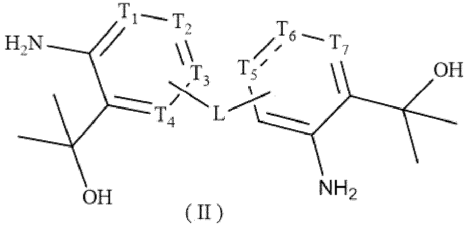

## Description

### TECHNICAL FIELD

The present invention belongs to the field of pharmaceutical preparations, and relates to ophthalmic formulations of pyridine phenyl compounds and preparation methods thereof, as well as application thereof in ophthalmic diseases.

### BACKGROUND ART

dry eye disease, also known as keratoconjunctivitis sicca, refers to a variety of diseases caused by abnormal quality or quantity of tears or abnormal dynamics, resulting in decreased tear film stability and accompanied by ocular discomfort (or) ocular surface tissue lesions. Specific discomfort symptoms include eye irritation, visual impairment, and unstable tear film. Some of the syndromes are caused by ocular surface inflammation, resulting in a loss of lacrimal gland function. In addition, it is also related to systemic autoimmunity.

Due to the production of toxic aldehydes such as malondialdehyde (MDA) and 4-hydroxy-2-nonenal (4HNE) through metabolic mechanisms in the body or ocular organs, these aldehydes highly react with proteins, carbohydrates, lipids, and DNA, leading to chemical modifications of biomolecules and activation of inflammatory molecule regulators such as NF-kappaB, thereby promoting damage to different organs. This is one of the causes of dry eye disease.

Through the present application' studies, the small molecule drugs are administrated into inflamed sites of eyes in the form of eye drops, through complexation reactions with endogenous aldehydes, therefore the toxicity of aldehydes is reduced, inflammation is lowered, and the effect of treating dry eye disease is achieved.

WO2020125659 discloses a pyridine phenyl aldehyde binding agent compound, and a series of compounds satisfying the formula I, including its isomers or pharmaceutically acceptable salts:

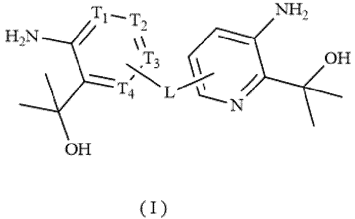

However, its ophthalmic formulations and their therapeutic effects on ophthalmic diseases have not yet been disclosed. Therefore, further research is needed to discover the efficacy and safety of ophthalmic formulations containing the above-mentioned small molecule compounds for ophthalmic diseases such as dry eye disease, allergic conjunctivitis, macular degeneration, cataracts, keratoconus, bullous keratopathy, Fuchs endothelial corneal dystrophy, ocular cicatricial pemphigoid, meibomian gland dysfunction, uveitis, scleritis, Stevens-Johnson syndrome, ocular rosacea, Sjögren syndrome, and to develop ophthalmic formulations with stable preparation process and quality.

### SUMMARY

A purpose of the present application is to provide a safe and effective ophthalmic formulation, which has a simple preparation process and stable quality, being suitable for industrial production.

The present application provides an ophthalmic formulation including an active ingredient, that is, pyridine phenyl compound, and an excipient, wherein the excipient includes solubilizing agent, pH modifier, osmotic pressure regulator, antimicrobial preservative, and antioxidant, the active compound includes a compound of formula (I), isomers thereof, or a pharmaceutically acceptable salt thereof. The present application provides a compound of formula (I) or a pharmaceutically acceptable salt thereof, 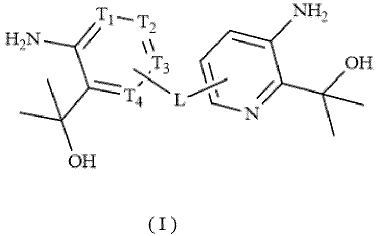 wherein,
T₁, T₂, T₃, and T₄ are independently selected from N, C, or CR₁, respectively;
L is selected from single bond,-O-,-S-,-NR₂- or -(CR₃R₄) n-;
R₁ is selected from H, F, Cl, Br, I, OH, or NH₂;
R₂ is selected from H and a C₁₋₃ alkyl group optionally substituted with 1, 2, or 3 Rₐ;
R₃ and R₄ are independently selected from H, F, Cl, Br, I, OH, NH₂, CN, or a C₁₋₃ alkyl group optionally substituted with 1, 2, or 3 R_{b} , respectively;
n is selected from 1, 2, or 3;
Rₐ and R_{b} are independently selected from H, F, Cl, Br, I, OH, NH₂, CN, or CH₃, respectively.

In some embodiments of the present application, the above-mentioned R₂ is selected from H, CH₃, or CH₂CH₃, wherein CH₃ and CH₂CH₃ are optionally substituted with 1, 2, or 3 Rₐ, and other variables are as defined in the present application.

In some embodiments of the present application, the above-mentioned R₂ is selected from H, CH₃, or CH₂CH₃, and other variables are as defined in the present application.

In some embodiments of the present application, the above-mentioned R₃ and R₄ are independently selected from H, F, Cl, Br, I, OH, NH₂, CN, CH₃, or CH₂CH₃, respectively, wherein CH₃ and CH₂CH₃ are optionally substituted with 1, 2, or 3 R_{b}. Other variables are as defined in the present application.

In some embodiments of the present application, the above-mentioned R₃ and R₄ are independently selected from H, F, Cl, Br, I, OH, NH₂, CN, CH₃, or CH₂CH₃, respectively. Other variables are as defined in the present application.

In some embodiments of the present application, the above-mentioned L is selected from single bond, -O-, -S-, -NH-, -(CH₂)₂- or -CH₂-, and other variables are as defined in the present application.

The present application provides a compound of formula (II) or a pharmaceutically acceptable salt thereof, 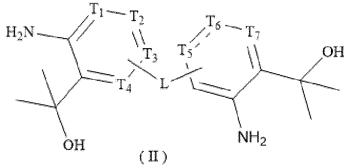 wherein,
The 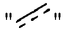 is selected from a single bond or a double bond;
T₁, T₂, T₃, and T₄ are independently selected from N, C, or CR₁, respectively;
T₅ is selected from C, CR₅, or C=O;
T₆ is selected from C, CR₆, or N;
T₇ is selected from N or CR₇;
When T₅ is selected from C=O and T₆ is selected from N, the " 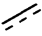 " is selected from a single bond;
L is selected from a single bond, -O-, -S-, -NR₂- or -(CR₃R₄) n-;
Each R₁ is independently selected from H, F, Cl, Br, I, OH, or NH₂, respectively;
R₂ is selected from H and a C₁₋₃ alkyl group optionally substituted with 1, 2, or 3 Rₐ;
R₃ and R₄ are independently selected from H, F, Cl, Br, I, OH, NH₂, CN, or a C₁₋₃ alkyl group optionally substituted with 1, 2, or 3 R_{b}, respectively;
R₅, R₆, and R₇ are independently selected from H, F, Cl, Br, or I, respectively;
n is selected from 1, 2, or 3;
Rₐ and R_{b} are independently selected from H, F, Cl, Br, I, OH, NH₂, CN, or CH₃, respectively.

In some embodiments of the present application, the above-mentioned R₂ is selected from H, CH₃, or CH₂CH₃, wherein CH₃ and CH₂CH₃ are optionally substituted with 1, 2, or 3 Rₐ, and other variables are as defined in the present application.

In some embodiments of the present application, the above-mentioned R₂ is selected from H, CH₃, or CH₂CH₃, and other variables are as defined in the present application.

In some embodiments of the present application, the above-mentioned R₃ and R₄ are independently selected from H, F, Cl, Br, I, OH, NH₂, CN, CH₃, or CH₂CH₃, respectively, wherein CH₃ and CH₂CH₃ are optionally substituted with 1, 2, or 3 Rb. Other variables are as defined in the present application.

In some embodiments of the present application, the above-mentioned R₃ and R₄ are independently selected from H, F, Cl, Br, I, OH, NH₂, CN, CH₃, or CH₂CH₃, respectively, and other variables are as defined in the present application.

In some embodiments of the present application, the above-mentioned L is selected from single bond, -O-, -S-, -NH-, -(CH₂)₂-, and-CH₂-, and other variables are as defined in the present application.

There are also some technical solutions of the present application that can be obtained by arbitrarily combining the above variables.

In some embodiments of the present application, the above-mentioned compound or its pharmaceutically acceptable salt is selected from: 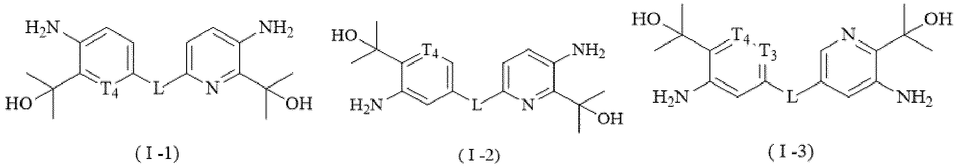 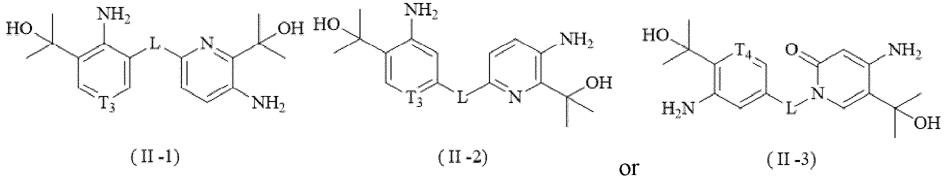 wherein,
T₃ and T₄ are independently selected from N and CR₁, respectively;
R₁ and L are as defined in the present application.

In some embodiments of the present application, the above-mentioned compound or its pharmaceutically acceptable salt is selected from: 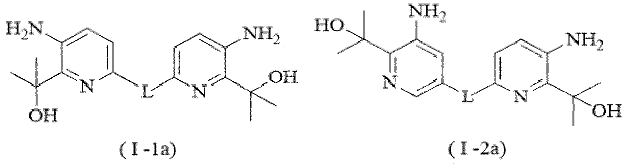 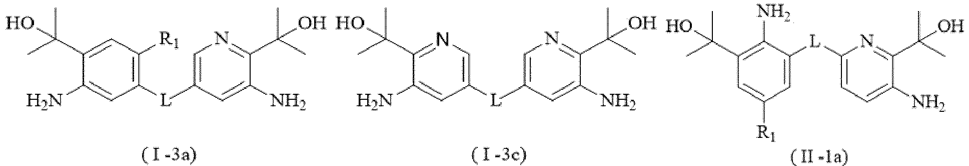 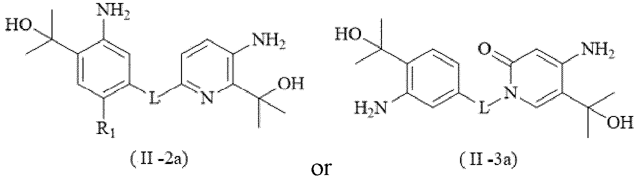 wherein,
R₁ and L are as defined in the present application.

In the present application, as one of the embodiments, the compound of formula (I) or its pharmaceutically acceptable salt is selected from:

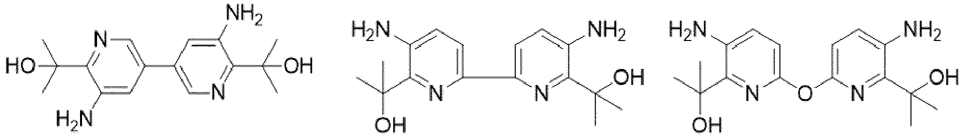

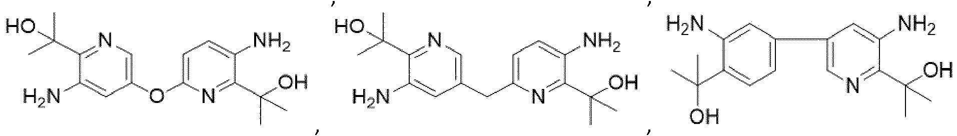

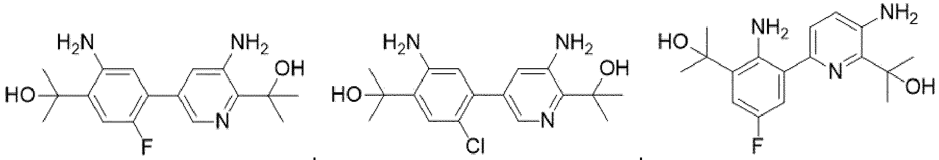

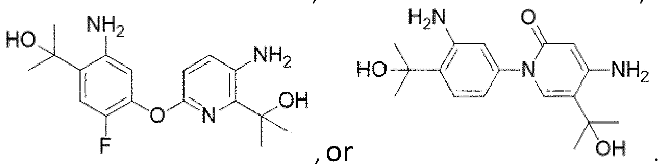

In the present application, as one of the embodiments, the compound of formula (III), its isomer, or its pharmaceutically acceptable salt is:

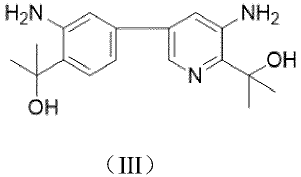

In the present application, as one of the embodiments, the compound of formula (IV), its isomer, or its pharmaceutically acceptable salt is:

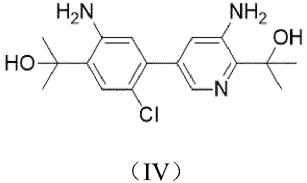

In the present application, as one of the embodiments, the compound of formula (V), its isomer, or its pharmaceutically acceptable salt is:

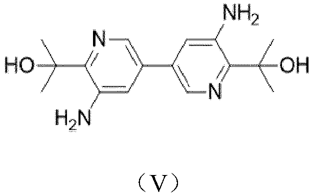

In the present application, as one of the embodiments, the compound of formula (VI), its isomer, or its pharmaceutically acceptable salt is:

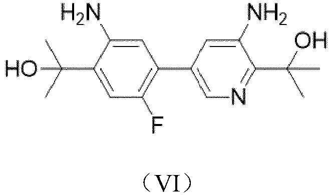

In the present application, as one of the embodiments, the compound of formula (VII), its isomer, or its pharmaceutically acceptable salt is:

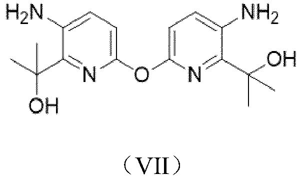

In the present application, as one of the embodiments, the compound of formula (VIII), its isomer, or its pharmaceutically acceptable salt is:

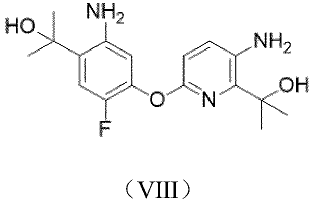

In the present application, as one of the embodiments, the compound of formula (III) further includes one water molecule, which is the following compound of formula IX, its isomer, or its pharmaceutically acceptable salt:

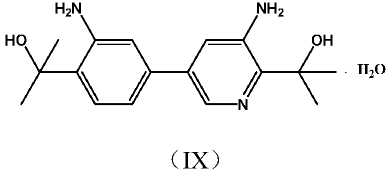

In the present application, as one of the embodiments, the ophthalmic formulation includes one of the compounds of formula (III), formula (IV), formula (V), formula (VI), formula (VII), formula (VIII), or formula (IX) disclosed in the present application.

In the present application, as one of the embodiments, the ophthalmic formulation includes one or more solubilizing agents.

In the present application, as one of the embodiments, the ophthalmic formulation includes one or more pH modifiers.

In the present application, as one of the embodiments, the ophthalmic formulation includes one or more osmotic pressure regulators.

In the present application, as one of the embodiments, the ophthalmic formulation includes one or more antimicrobial preservatives.

In the present application, as one of the embodiments, the ophthalmic formulation includes one or more antioxidants.

In the present application, as one of the embodiments, the solubilizing agent is selected from methylated-β-cyclodextrin (RM-β-CD), hydroxypropyl betadex (HP-β-CD), hydroxypropyl-γ-cyclodextrin (HP-γ-CD), sulfobutylether-β-cyclodextrin (SBE-β-CD), poloxamer 407, Polysorbate 80, povidone (PVP), polyethylene glycol (PEG400), or a combination of two or more of them.

In the present application, as one of the embodiments, the pH modifiers is selected from monobasic sodium phosphate monohydrate, dibasic sodium phosphate, borax, boric acid, citric acid dihydrate, hydrochloric acid, sodium hydroxide, or a combination of two or more of them.

In the present application, as one of the embodiments, the osmotic pressure regulator is selected from sodium chloride, boric acid, borax, glucose, mannitol, or a combination of two or more of them.

In the present application, as one of the embodiments, the antimicrobial preservative is selected from benzalkonium chloride, chlorhexidine acetate, phenylmercury acetate, or a combination of two or more of them.

In the present application, as one of the embodiments, the antioxidant is selected from butylated hydroxyanisole (BHA), vitamin E (VE), or a combination of two or more of them.

In the present application, as one of the embodiments, the freeze-drying solvent is selected from 95% ethanol, tert-butanol, isopropanol, or acetonitrile.

The present application provides an ophthalmic formulation including an active ingredient pyridine phenyl compound, one or more solubilizing agents, one or more pH modifiers, one or more osmotic pressure regulators, one or more antimicrobial preservatives, and one or more antioxidants, wherein the pyridine phenyl compound includes the compounds of formula (III), formula (IV), formula (V), formula (VI), formula (VII), formula VIII or formula (IX), its isomers or its pharmaceutically acceptable salts.

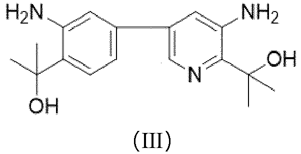

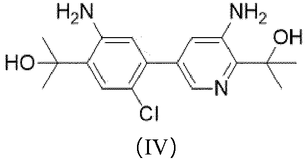

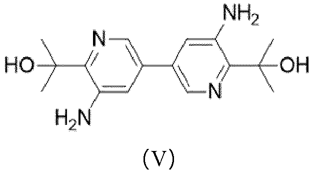

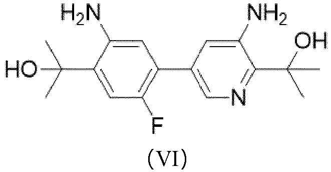

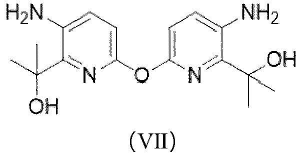

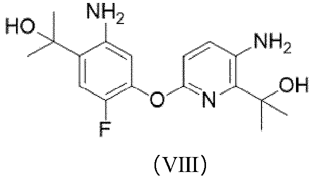

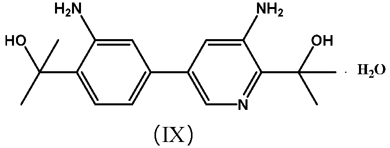

In the ophthalmic formulation provided in the present application, the content of the active ingredient is 0.05-0.6% w/v, preferably 0.1% w/v, 0.11% w/v, 0.12% w/v, 0.13% w/v, 0.14% w/v, 0.15% w/v, 0.16% w/v, 0.17% w/v, 0.18% w/v, 0.19% w/v, 0.2% w/v, 0.21% w/v, 0.22% w/v, 0.23% w/v, 0.24% w/v, 0.25% w/v, 0.26% w/v, 0.27% w/v, 0.28% w/v, 0.29% w/v, 0.3% w/v, 0.31% w/v, 0.32% w/v, 0.33% w/v 0.34% w/v, 0.35% w/v, 0.36% w/v, 0.37% w/v, 0.38% w/v, 0.39% w/v, 0.4% w/v, 0.41% w/v, 0.42% w/v, 0.43% w/v, 0.44% w/v, 0.45% w/v, 0.46% w/v, 0.47% w/v, 0.48% w/v, 0.49% w/v, 0.5% w/v, 0.51% w/v, 0.52% w/v, 0.53% w/v, 0.54% w/v, or 0.55% w/v.

In the ophthalmic formulation provided in the present application, the solubilizing agent is selected from methylated-β-cyclodextrin (RM-β-CD), hydroxypropyl betadex (HP-β-CD), hydroxypropyl-γ-cyclodextrin (HP-γ-CD), sulfobutylether-β-cyclodextrin (SBE-β-CD), poloxamer 407, Polysorbate 80, povidone (PVP), polyethylene glycol (PEG400), or a combination of two or more of them.

In the ophthalmic formulation provided in the present application, the content of the solubilizing agent is 0.2% to 15% w/v, preferably 0.3% w/v, 0.4% w/v, 0.5% w/v, 0.6% w/v, 0.65% w/v, 0.7% w/v, 0.75% w/v, 0.8% w/v, 0.85% w/v, 0.9% w/v, 0.95% w/v, 1.0% w/v, 1.1% w/v, 1.2% w/v, 1.3% w/v, 1.4% w/v, 1.5% w/v, 1.55% w/v, 1.6% w/v, 1.65% w/v, 1.7% w/v, 1.75% w/v, 1.8% w/v, 1.9% w/v, 2.0% w/v 2.1% w/v, 2.2% w/v, 2.3% w/v, 2.4% w/v, 2.5% w/v, 2.6% w/v, 2.7% w/v, 2.8% w/v, 2.9% w/v, 3.0% w/v, 3.3% w/v, 3.5% w/v, 3.6% w/v, 3.8% w/v, 4.0% w/v, 4.2% w/v, 4.4% w/v, 4.5% w/v, 4.6% w/v, 4.8% w/v, 5.0% w/v, 5.2% w/v, 5.4% w/v, 5.5% w/v, 5.6% w/v, 5.8% w/v, 6.0% w/v, 6.2% w/v, 6.5% w/v, 6.8% w/v 7.0% w/v, 7.2% w/v, 7.4% w/v, 7.5% w/v, 7.6% w/v, 7.8% w/v, 8.0% w/v, 8.2% w/v, 8.5% w/v, 8.6% w/v 8.8% w/v, 9.0% w/v, 9.2% w/v, 9.4% w/v, 9.5% w/v, 9.6% w/v, 9.8% w/v, or 9.9% w/v.

In the ophthalmic formulation provided in the present application, the content of the solubilizing agent hydroxypropyl betadex (HP-β-CD) is 0.5% w/v~12% w/v, preferably 0.7% w/v~10% w/v, more preferably 1% w/v~8% w/v, most preferably 1.2% w/v, 1.7% w/v, 1.75% w/v, 2% w/v, 2.5% w/v, 2.8% w/v, 3% w/v, 3.3% w/v, 3.5% w/v, 4% w/v, 4.4% w/v, 5% w/v, 5.5% w/v, 6% w/v, 7% w/v, 7.5% w/v, or 8% w/v.

In the ophthalmic formulation provided by the present application, the content of sulfobutylether-β-cyclodextrin (SBE-β-CD) is selected from 4% to 13%, preferably 4.3% w/v, 5% w/v, 6% w/v, 7% w/v, 8% w/v, 9% w/v, 10% w/v, 11% w/v, 12% w/v, or 12.5% w/v.

In the ophthalmic formulation provided in the present application, the content of the pH modifier is 0.12-20% w/v, preferably 0.2% w/v, 0.25% w/v, 0.3% w/v, 0.35% w/v, 0.4% w/v, 0.47% w/v, 0.5% w/v, 0.6% w/v, 0.7% w/v, 0.8% w/v, 0.81% w/v, 0.9% w/v, 1% w/v, 2% w/v, 3% w/v, 4% w/v, 5% w/v, 6% w/v, 7% w/v, 8% w/v, 9% w/v, 10% w/v, 11% w/v, 12% w/v, 13% w/v, 14% w/v, 15%. w/v, 16% w/v, 17% w/v, 18% w/v, 19% w/v, or 20% w/v.

In the ophthalmic formulation provided in the present application, the content of the pH modifier monobasic sodium phosphate monohydrate is 0.1% w/v~0.5% w/v, preferably 0.12% w/v~0.45% w/v, more preferably 0.15% w/v, 0.2% w/v, 0.25% w/v, 0.3% w/v, 0.35% w/v, or 0.4% w/v.

In the ophthalmic formulation provided in the present application, the content of pH modifier dibasic sodium phosphate is 0.3% w/v~1% w/v, preferably 0.4% w/v~0.9% w/v, more preferably 0.45% w/v, 0.47% w/v, 0.5% w/v, 0.55% w/v, 0.6% w/v, 0.65% w/v, 0.7% w/v, 0.75% w/v, or 0.81% w/v.

In the ophthalmic formulation provided in the present application, the content of the osmotic pressure regulator is 0.1%~1% w/v, preferably 0.2% w/v, 0.21% w/v, 0.22% w/v, 0.23% w/v, 0.24% w/v, 0.25% w/v, 0.26% w/v, 0.27% w/v, 0.28% w/v, 0.29% w/v, 0.3% w/v, 0.31% w/v, 0.32% w/v, 0.33% w/v, 0.34% w/v, 0.35% w/v, 0.36% w/v, 0.37% w/v, 0.38% w/v, 0.39% w/v, 0.4% w/v, 0.42% w/v, 0.44% w/v, 0.45% w/v 0.46% w/v, 0.48% w/v, 0.5% w/v, 0.55% w/v, 0.6% w/v, 0.65% w/v, 0.7% w/v, 0.75% w/v, 0.8% w/v, 0.85% w/v, 0.9% w/v, or 0.95% w/v.

In the ophthalmic formulation provided in the present application, the content of the osmotic pressure regulator sodium chloride is 0.2% w/v~0.8% w/v, preferably 0.25% w/v~0.7% w/v, more preferably 0.26% w/v, 0.27% w/v, 0.3% w/v, 0.34% w/v, 0.36% w/v, 0.4% w/v, 0.45% w/v, 0.5% w/v, 0.55% w/v, 0.6% w/v, or 0.65% w/v.

In the ophthalmic formulation provided in the present application, the content of the antimicrobial preservative is 0.001% w/v~0.02% w/v, preferably 0.002% w/v~0.018% w/v, further preferably 0.003% w/v~0.016% w/v, most preferably 0.004% w/v, 0.0045% w/v, 0.005% w/v, 0.0055% w/v, 0.006% w/v, 0.0065% w/v, 0.007% w/v, 0.0075% w/v, 0.008% w/v, 0.0085% w/v, 0.009% w/v, 0.01% w/v, 0.012% w/v, 0.014% w/v, or 0.015% w/v.

In the ophthalmic formulation provided by the present application, the content of the antimicrobial preservative benzalkonium chloride is 0.001% w/v~0.02% w/v, preferably 0.002% w/v~0.018% w/v, further preferably 0.003% w/v~0.016% w/v, most preferably 0.004% w/v, 0.0045% w/v, 0.005% w/v, 0.0055% w/v, 0.006% w/v, 0.0065% w/v, 0.007% w/v, 0.0075% w/v, 0.008% w/v, 0.0085% w/v, 0.009% w/v, 0.01% w/v, 0.012% w/v, 0.014% w/v, or 0.015% w/v.

In the ophthalmic formulation provided by the present application, the content of the antimicrobial preservative chlorhexidine acetate is 0.001% w/v~0.02% w/v, preferably 0.002% w/v~0.018% w/v, further preferably 0.003% w/v~0.016% w/v, most preferably 0.004% w/v, 0.0045% w/v, 0.005% w/v, 0.0055% w/v, 0.006% w/v, 0.0065% w/v, 0.007% w/v, 0.0075% w/v, 0.008% w/v, 0.0085% w/v, 0.009% w/v, 0.01% w/v, 0.012% w/v, 0.014% w/v, or 0.015% w/v.

In the ophthalmic formulation provided in the present application, the content of the antioxidant is 0.1% to 0.8% w/v, preferably 0.2% w/v, 0.3% w/v, 0.4% w/v, 0.5% w/v, 0.6% w/v, or 0.7% w/v.

In the ophthalmic formulation provided by the present application, the ophthalmic formulation includes: a compound of formula (IX) and its isomers or its pharmaceutically acceptable salts, with a content of 0.1% w/v~0.5% w/v; further includes excipient: hydroxypropyl betadex (HP-β-CD), with a content of 0.7% w/v~3.5% w/v, preferably 1.75% w/v, 3.5% w/v, or 0.7% w/v; dibasic sodium phosphate hydrogen phosphate, with a content of 0.81% w/v; monobasic sodium phosphate monohydrate, with a content of 0.12% w/v; sodium chloride, with a content of 0.25% w/v~0.45% w/v, preferably 0.27% w/v, 0.36% w/v, or 0.4% w/v; and chlorhexidine acetate, with a content of 0.01% w/v.

In the ophthalmic formulation provided in the present application, the pH range of the ophthalmic formulation is 5.0~9.0, preferably 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 6.91, 6.92, 6.93, 6.94, 6.95, 6.96, 6.97, 6.98, 6.99, 7, 7.1, 7.2, 7.3, 7.4, 7.5, 7.6, 7.7, 7.72, 7.73, 7.74, 7.75, 7.76, 7.77, 7.8, 7.9, 8.0, 8.1, 8.2, 8.3, 8.4, 8.5, 8.53, 8.54, 8.55 8.56, 8.57, 8.58, 8.59, 8.6, 8.7, 8.8, or 8.9.

The preparation method of the ophthalmic formulation provided in the present application includes rotary evaporation process, concentration dilution method, and freeze-drying method.

Through the study of stability, the ophthalmic formulation in the present application is measured in terms of changes in content and relevant substances. The results show that the ophthalmic formulation provided by the present application is table in nature, with no significant differences in relevant substances and contents.

The dosage form of the ophthalmic formulation provided by the present application can be liquid formulation, for example, eye drop, eyewash or intraocular injection solution; ophthalmic semisolid formulation, for example, eye ointment, eye cream, or eye gel; and ophthalmic solid formulation, for example, eye film, eye pellet, and intraocular insert.

The present application provides an ophthalmic formulation for use in the preparation of drugs for treating ophthalmic diseases, preferably for dry eye disease, allergic conjunctivitis, macular degeneration, cataracts, keratoconus, bullous keratopathy, Fuchs endothelial corneal dystrophy, ocular cicatricial pemphigoid, meibomian gland dysfunction, uveitis, scleritis, Stevens-Johnson syndrome, ocular rosacea, or Sjögren syndrome.

The therapeutic effect of the ophthalmic formulation provided by the present application is investigated by inducing dry eye disease model in C57BL/6 mice by subcutaneous injection of scopolamine hydrobromide solution into the lower limbs, and inducing dry eye disease model in SD rats by eye drops of hypertonic sodium chloride solution. The results show that the ophthalmic formulation of the present application can improve tear secretion volume and corneal damage in dry eye disease model mice. Through pharmacokinetic studies in the eyes of New Zealand rabbits, it has been found that the active ingredients have a high concentration distribution in the corneal and conjunctival tissues of the ocular surface, which is beneficial for the treatment of eye diseases, and the concentration distribution in the fundus is low, which indicate a lower risk of causing adverse reactions in the fundus.

### Definition and Explanation

Unless otherwise specified, the following terms and phrases used herein are intended to have the meanings set forth below. A specific term or phrase should not be regarded as indefinite or unclear in the absence of a special definition, but should be understood according to its ordinary meaning. When trade names appear herein, they are intended to refer to the corresponding products or their active ingredients. The term "pharmaceutically acceptable" as used here refers to those compounds, materials, compositions, and/or dosage forms that, within the scope of sound medical judgment, are suitable for use in contact with the tissues of humans and animals without undue toxicity, irritation, allergic reactions, or other problems or complications, commensurate with a reasonable benefit/risk ratio.

The term "pharmaceutically acceptable salt" refers to a salt of the compound of the present application, prepared from compounds with specific substituents discovered by the present application and relatively non-toxic acids or bases. When the compounds of the present application contain relatively acidic functional groups, base addition salts can be obtained by contacting the neutral form of such compounds with a sufficient amount of the base in a pure solution or a suitable inert solvent. Pharmaceutically acceptable base addition salts include sodium, potassium, calcium, ammonium, organic amine, or magnesium salts, or similar salts. When the compounds of the present application contain relatively basic functional groups, acid addition salts can be obtained by contacting the neutral form of such compounds with a sufficient amount of the acid in a pure solution or a suitable inert solvent. Examples of pharmaceutically acceptable acid addition salts include inorganic acid salts, such as hydrochloric acid, hydrobromic acid, nitric acid, carbonic acid, bicarbonate, phosphoric acid, monohydrogen phosphate, dihydrogen phosphate, sulfuric acid, bisulfate, hydroiodic acid, phosphorous acid, etc.; and organic acid salts, said organic acids include, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, methanesulfonic acid, and similar acids; also including salts of amino acids (such as arginine, etc.), and salts of organic acids such as glucuronic acid. Certain specific compounds of the present application contain both basic and acidic functional groups, allowing them to be converted into either base or acid addition salts.

The pharmaceutically acceptable salts of the present application can be synthesized from parent compounds containing acidic or basic groups through conventional chemical methods. Generally, such salts are prepared by reacting the free acid or base forms of these compounds with a stoichiometrically appropriate base or acid in water, an organic solvent, or a mixture of both.

In addition to salt forms, the compounds provided by the present application are also present in prodrug forms. The prodrugs of the compounds described herein readily undergo chemical changes under physiological conditions to be converted into the compounds of the present application. Furthermore, prodrugs can be converted into the compounds of the present application through chemical or biochemical methods in vivo.

Certain compounds of the present application may present in non-solvated forms or solvated forms, including hydrated forms. Generally, solvated forms are equivalent to non-solvated forms and are included within the scope of the present application.

Optically active (R)- and (S)-isomers, as well as D and L isomers, can be prepared through chiral synthesis, chiral reagents, or other conventional techniques. If a single enantiomer of a compound of the present application is desired, it can be prepared by asymmetric synthesis or derivatization with a chiral auxiliary, where the resulting diastereomeric mixture is separated, and the auxiliary group is cleaved to provide the pure desired enantiomer. Alternatively, when the molecule contains a basic functional group (such as an amino group) or an acidic functional group (such as a carboxyl group), non-diastereomeric salts can be formed with an appropriate optically active acid or base, followed by conventional methods known in the art for diastereomeric resolution, and then the pure enantiomer can be recovered. Additionally, the separation of enantiomers and diastereomers is usually accomplished using chromatography with a chiral stationary phase, optionally combined with chemical derivatization (e.g., formation of carbamates from amines). The compounds of the present application may contain non-natural proportions of atomic isotopes at one or more of the atoms constituting the compound. For example, compounds can be labeled with radioactive isotopes, such as tritium (3H), iodine-125 (125I), or carbon-14 (14C). For example, hydrogen can be replaced with deuterium to form deuterated drugs, where the carbon-deuterium bond is stronger than the carbon-hydrogen bond, offering advantages such as reduced toxic side effects, increased drug stability, enhanced efficacy, and prolonged biological half-life compared to non-deuterated drugs. All isotopic variations of the compounds of the present application, whether radioactive or not, are included within the scope of the present application.

The term "optional" or "optionally" means that the subsequently described event or circumstance may but is not necessarily required to occur, and the description includes situations where the event or circumstance occurs and situations where it does not.

The term "substituted" means that any one or more hydrogen atoms on a specific atom are replaced by a substituent, which may include deuterium and variants of hydrogen, provided that the valence of the specific atom is normal and the resulting compound is stable. When the substituent is oxygen (i.e., =O), it means that two hydrogen atoms are replaced. Oxygen substitution does not occur on aromatic groups. The term "optionally substituted" means that it may be substituted or unsubstituted, and unless otherwise specified, the type and number of substituents can be determined arbitrarily on a chemically realizable basis.

When any variable (e.g., R) appears more than once in the composition or structure of a compound, its definition in each case is independent. Thus, for example, if a group is substituted by 0-2 R groups, the group may optionally be substituted by up to two R groups, and in each case, R has independent options. Additionally, combinations of substituents and/or their variants are permitted only if such combinations result in stable compounds.

When the number of a linking group is 0, such as -(CRR)₀-, it means that the linking group is a single bond.

When one of the variables is selected from a single bond, it means that the two groups to which it is connected are directly linked, such as in A-L-Z where L represents a single bond, meaning the structure is actually A-Z.

When a substituent is absent, it means that the substituent does not exist, such as in A-X where X is absent, meaning the structure is actually A. When the listed substituents do not specify through which atom they are connected to the substituted group, the substituent can be bonded through any of its atoms, for example, a pyridyl group as a substituent can be connected to the substituted group through any carbon atom on the pyridine ring. When the listed linking groups do not specify their direction of connection, the direction of connection is arbitrary, for example, in the linking group 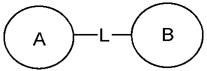 L being -M-W-, -M-W- can connect ring A and ring B to form 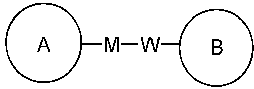 in the same direction as the reading order from left to right, or form 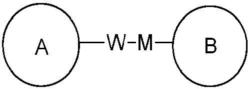 in the opposite direction. Combinations of linking groups, substituents, and/or their variants are permitted only if such combinations result in stable compounds.

Unless otherwise specified, the term "C₁₋₆ alkyl" is used to represent a straight or branched saturated hydrocarbon group consisting of 1 to 6 carbon atoms. The C₁₋₆ alkyl includes C₁₋₅, C₁₋₄, C₁₋₃, C₁₋₂, C₂₋₆, C₂₋₄, C₆, and C₅ alkyl, etc.; it can be monovalent (such as methyl), divalent (such as methylene), or polyvalent (such as methine). Examples of C₁₋₆ alkyl include, but not limited to, methyl (Me), ethyl (Et), propyl (including n-propyl and isopropyl), butyl (including n-butyl, isobutyl, s-butyl, and t-butyl), pentyl (including n-pentyl, isopentyl, and neopentyl), hexyl, etc.

Unless otherwise specified, the term "C₁₋₃ alkyl" is used to represent a straight or branched saturated hydrocarbon group consisting of 1 to 3 carbon atoms. The C₁₋₃ alkyl includes C₁₋₂ and C₂₋₃ alkyl, etc.; it can be monovalent (such as methyl), divalent (such as methylene), or polyvalent (such as methine). Examples of C₁₋₃ alkyl include, but not limited to, methyl (Me), ethyl (Et), propyl (including n-propyl and isopropyl), etc.

Unless otherwise specified, the term "C₁₋₃ alkoxyl" represents those alkyl groups containing 1 to 3 carbon atoms that are connected to the rest of the molecule through an oxygen atom. The C₁₋₃ alkoxyl includes C₁₋₂, C₂₋₃, C₃, and C₂ alkoxyl, etc. Examples of C₁₋₃ alkoxyl include, but not limited to, methoxyl, ethoxyl, propoxyl (including n-propoxyl and iso-propoxyl), etc.

Unless otherwise specified, Cₙ₋ₙ₊ₘ or Cₙ₋Cₙ₊ₘ includes any specific case from n to n+m carbons, for example, C₁₋₁₂ includes C₁, C₂, C₃, C₄, C₅, C₆, C₇, C₈, C₉, C₁₀, C₁₁, and C₁₂, and also includes any range within n to n+m, for example, C₁₋₁₂ includes C₁₋₃, C₁₋₆, C₁₋₉, C₃₋₆, C₃₋₉, C₃₋₁₂, C₆₋₉, C₆₋₁₂, and C₉₋₁₂, etc.; similarly, n-membered to n+m-membered rings indicate rings with n to n+m atoms, for example, 3-12 membered rings include 3-membered, 4-membered, 5-membered, 6-membered, 7-membered, 8-membered, 9-membered, 10-membered, 11-membered, and 12-membered rings, and also include any range within n to n+m, for example, 3-12 membered rings include 3-6 membered, 3-9 membered, 5-6 membered, 5-7 membered, 6-7 membered, 6-8 membered, and 6-10 membered rings, etc.

The term "leaving group" refers to a functional group or atom that can be replaced by another functional group or atom through a substitution reaction (e.g., nucleophilic substitution). For example, representative leaving groups include triflates; chloro, bromo, iodo; sulfonates, such as mesylate, tosylate, brosylate, p-toluenesulfonate, etc.; acyloxy, such as acetoxy, trifluoroacetoxy, etc.

The term "protecting group" includes, but not limited to, "amino protecting group," "hydroxy protecting group," or "thiol protecting group." The term "amino protecting group" refers to a protecting group suitable for preventing side reactions at the nitrogen of an amino group. Representative amino protecting groups include, but not limited to, formyl; acyl, such as alkanoyl (e.g., acetyl, trichloroacetyl, or trifluoroacetyl); alkoxycarbonyl, such as tert-butoxycarbonyl (Boc); aryloxycarbonyl, such as benzyloxycarbonyl (Cbz) and 9-fluorenylmethoxycarbonyl (Fmoc); arylmethyl, such as benzyl (Bn), trityl (Tr), 1,1-di-(4'-methoxyphenyl)methyl; silyl, such as trimethylsilyl (TMS) and tert-butyldimethylsilyl (TBS), etc. The term "hydroxy protecting group" refers to a protecting group suitable for preventing side reactions of a hydroxyl group. Representative hydroxy protecting groups include, but not limited to, alkyl, such as methyl, ethyl, and tert-butyl; acyl, such as alkanoyl (e.g., acetyl); arylmethyl, such as benzyl (Bn), p-methoxybenzyl (PMB), 9-fluorenylmethyl (Fm), and diphenylmethyl (benzhydryl, DPM); silyl, such as trimethylsilyl (TMS) and tert-butyldimethylsilyl (TBS), etc.

The compounds of the present application can be prepared by various synthetic methods known to those skilled in the art, including the specific embodiments listed below, their combination with other chemical synthesis methods, and equivalent alternatives known to those skilled in the art. Preferred embodiments include, but not limited to, the examples of the present application.

The solvents used in the present application are commercially available. The following abbreviations are used in the present application: aq represents water; HATU represents O-(7-azabenzotriazol-1-yl)-N,N,N',N'-tetramethyluronium hexafluorophosphate; EDC represents N-(3-dimethylaminopropyl)-N'-ethylcarbodiimide hydrochloride; m-CPBA represents 3-chloroperoxybenzoic acid; eq represents equivalent; CDI represents carbonyl diimidazole; DCM represents dichloromethane; PE represents petroleum ether; DIAD represents diisopropyl azodicarboxylate; DMF represents N,N-dimethylformamide; DMSO represents dimethyl sulfoxide; EtOAc represents ethyl acetate; EtOH represents ethanol; MeOH represents methanol; CBz represents benzyloxycarbonyl, an amine protecting group; BOC represents tert-butoxycarbonyl, an amine protecting group; HOAc represents acetic acid; NaCNBH₃ represents sodium cyanoborohydride; r.t. represents room temperature; O/N represents overnight; THF represents tetrahydrofuran; Boc₂O represents di-tert-butyl dicarbonate; TFA represents trifluoroacetic acid; DIPEA represents diisopropylethylamine; SOCl₂ represents thionyl chloride; CS₂ represents carbon disulfide; TsOH represents p-toluenesulfonic acid; NFSI represents N-fluoro-N-(phenylsulfonyl)benzenesulfonamide; NCS represents N-chlorosuccinimide; n-Bu4NF represents tetrabutylammonium fluoride; iPrOH represents isopropanol; mp represents melting point; LDA represents lithium diisopropylamide; LiHMDS represents lithium hexamethyldisilazide; Xantphos represents 4,5-bis(diphenylphosphino)-9,9-dimethylxanthene; LiAlH4 represents lithium aluminum hydride; Pd(dba)2 represents tris(dibenzylideneacetone)dipalladium; mCPBA represents meta-chloroperoxybenzoic acid; pd(dppf)Cl2 represents [1,1'-bis(diphenylphosphino)ferrocene]dichloropalladium; DBU represents 1,8-diazabicyclo[5.4.0]undec-7-ene.

Compounds are named according to conventional naming principles in this field or using ChemDraw^{®} Software naming, and commercially available compounds use supplier catalog names.

### BRIEF DESCRIPTION OF THE DRAWINGS

FIG. 1: XRPD spectrum of compound of formula (IX) using Cu-Kα radiation.
FIG. 2: DSC spectrum of compound of formula (IX).
FIG. 3: TGA spectrum of compound of formula (IX).
FIG. 4: Tear secretion volume in a mouse model of dry eye disease for Examples 105, 106, and 107.
FIG. 5: Corneal fluorescein staining scores in a mouse model of dry eye disease for Examples 105, 106, and 107.
FIG. 6: Tear secretion volume in a rat model of dry eye disease for Examples 105 and 107.
FIG. 7: Corneal fluorescein staining scores in a rat model of dry eye disease for Examples 105 and 107.
FIG. 8: Tear film break-up time in a rat model of dry eye disease for Examples 105 and 107.
FIG. 9: Chemosis scores.
FIG. 10: Conjunctival hyperemia scores.
FIG. 11: Pathological results of H&E staining.

### DETAILED DESCRIPTION

The present application will be further illustrated in detail through the following examples. These examples are for illustrative purposes only, not intended to limit the scope of the present application.

### Examples 1-11

The active compound formula (IX) of the present application is an insoluble or almost insoluble compound in water. Examples 1-11 were prepared according to the specific processes in Table 1. The results showed that the compound of formula (IX) still could not be dissolved after the different solubilizing agent, heating etc., operations in Examples 1, 4, 5, 6, 7, 9, and 10. The solubilizing agent used in Examples 2, 3, 8, and 11 have a certain promoting effect on solubility.

**Table 1**

| Examples | Operation process |
|---|---|
| 1 | Adding 50 mg of compound (IX) to 10 mL of water, heating in a 50°C water bath for 2 hours, adding 6 mL of 0.1M HCl and 1 mL of concentrated hydrochloric acid, remained insoluble, and again heating in a 50°C water bath for 2 hours, still remained insoluble. |
| 2 | Adding 50 mg of compound of formula (IX) to 10 mL of pH 6.8 phosphate solution, heating in a 50°C water bath for 2 hours, then grinding, adding 1 mL of concentrated hydrochloric acid, remained insoluble, and again heating in a 50°C water bath for 2 hours, achieving dissolution with opalescence. |
| 3 | Adding 50 mg of compound of formula (IX) to 10 mL of pH 6.8 phosphate solution, adding 0.5 g of HP-β-CD, grinding, remained insoluble, adding 0.5 g HP-β-CD , remained insoluble, and heating at 50°C for 2 hours, with a small amount remaining undissolved. |
| 4 | Adding 50 mg of compound of formula (IX) to 10 mL of pH 6.8 phosphate solution, remained insoluble, adding 2 mL of 10% PVP solution, grinding, remained insoluble, and heating in a 50°C water bath for 2 hours, still remained insoluble. |
| 5 | Adding 50 mg of compound of formula (IX) to 10 mL of pH 6.8 phosphate solution, remained insoluble, adding 0.2 mL PEG400, grinding, remained insoluble, and heating in a 50°C water bath for 2 hours, still remained insoluble. |
| 6 | Adding 50 mg of compound of formula (IX) to 10 mL of pH 7.4 phosphate solution, grinding, remained insoluble, and adding 10 mL of 0.3% poloxamer 407, remained insoluble, and heating in a 50°C water bath for 2 hours, still remained insoluble. |
| 7 | Adding 50 mg of compound (IX) to 10 mL of 0.3% poloxamer 407 solution, grinding, remained insoluble, adding 0.5 mL of Polysorbate 80, grinding, remained insoluble, and heating at 50°C for 2 hours, still remained insoluble. |
| 8 | Adding 0.5 mL PEG400 to 50mg of active compound of formula (IX), grinding, being partially dissolved, adding 0.5 mL PEG400, grinding, being dissolved, adding 1 mL of pH 6.8 phosphate solution in 9 portions to dissolve the active compound, and heated in a 50°C water bath, with flocculent precipitate. |
| 9 | Adding 1 mL of propylene glycol to 50 mg of active compound of formula (IX), grinding, remained insoluble, adding 9 mL of pH 6.8 phosphate solution, remained insoluble, and heating in a 50°C water bath, still remained insoluble. |
| 10 | Adding 1 mL of glycerol to 50 mg of active compound of formula (IX), grinding, remained insoluble, adding 9 mL of pH 6.8 phosphate solution, remained insoluble, and heating in a 50°C water bath, still remained insoluble. |
| 11 | Adding 7.5 mL of 95% ethanol to 50 mg of compound of formula (IX) and 2.5 mL of Polysorbate 80, being dissolved, adding water until the concentration of the active compound was 5 mg/mL, and left to settle for 1 hour with precipitate. |

### Examples 12-30

According to the prescriptions in Table 2, the active compound of formula (IX), propylene glycol, PEG400, Polysorbate 80 and other excipients were weighed and placed in a vial, and the active compound was sonicated to be dissolved in the above excipients to obtain a concentrated solution. The concentrated solution was dropwise added to the prescribed amount of medium. Examples 12 to 30 were obtained. The dissolution of the active compound was observed. The specific data is shown in Table 2. In Examples 20-23, a relatively stable solution of 5 mg/mL active compound was obtained.

**Table 2**

| Examples | Compound of Formula (IX) (mg) | Propylene Glycol (mg) | PEG400 (mg) | Polysorbate 80 (mg) | Medium (mL) | Dissolved state |
|---|---|---|---|---|---|---|
| 12 | 20 | 150 | / | 150 | 4 (0.2%HA solution) | Dissolved, left overnight with precipitation |
| 13 | 20 | 100 | / | 100 | 4 (0.2%HA solution) | Indissovable |
| 14 | 20 | 150 | / | 100 | 4 (0.2%HA solution) | Dissolved, left overnight with precipitation |
| 15 | 20 | 100 | 100 | 100 | 4 (0.2%HA solution) | Dissolved, left overnight with precipitation |
| 16 | 50 | 200 | 200 | 200 | 10 (0.2%HA solution) | Dissolved, left overnight with precipitation |
| 17 | 50 | 100 | 300 | 100 | 10 (0.2%HA solution) | Dissolved, left overnight with precipitation |
| 18 | 40 | 200 | 200 | 200 | 10 (0.2%HA solution) | Small amount of precipitate |
| 19 | 40 | 100 | 300 | 100 | 10 (0.2%HA solution) | Dissolved |
| 20 | 50 | 100 | 300 | 100 | 10 (10%PVP solution) | Dissolved |
| 21 | 50 | 100 | 300 | 100 | 10 (1%PVP solution) | Dissolved |
| 22 | 50 | 100 | 300 | 100 | 10 (3%PVP solution) | Dissolved |
| 23 | 50 | 100 | 300 | 100 | 10 (5%PVP solution) | Dissolved |
| 24 | 50 | / | 300 | / | 10 (water) | Undissolved |
| 25 | 50 | / | 300 | 100 | 10 (water) | Undissolved |
| 26 | 50 | / | 300 | | 10 (5%PVP solution) | Undissolved |
| 27 | 50 | / | 300 | 100 | 10 (5%PVP solution) | Undissolved |
| 28 | 50 | 100 | 300 | 100 | 10 (5%PVP) pH6.8 phosphate | Precipitate formed after 3h at room temperature |
| 29 | 50 | 100 | 300 | 100 | 10 (5%PVP) pH6.8 phosphate | Becoming turbid after staying at 5°C overnight |
| 30 | 50 | / | 300 | 100 | 10 (5%PVP) pH6.8 phosphate | Dissolved, with no precipitation after 6h at room temperature and precipitates after staying overnight |

### Examples 31-41

According to Table 3, the active compound of formula (IX) and HP-β-CD were dissolved in 95% ethanol. The above solution was then placed in rotary evaporation to dry the solvent, resulting in inclusion complex solid. The solid was dissolved in purified water, and a 5 mg/mL solution was prepared using purified water/pH6.8 phosphate medium based on the amount of active compound added. The dissolution was observed. The specific results are shown in Table 3.

**Table 3**

| Examples | Compound of Formula (IX) (mg) | HP-β-CD (mg) | 95% ethanol (mg) | Tert-butanol (mg) | Isopropanol (mg) | Water (mg) | Acetonitrile (mg) | Dissolution in (mL) medium (Active compound, 5 mg/mL) |
|---|---|---|---|---|---|---|---|---|
| 31 | 90 | 600 | 15 | / | / | / | / | Soluble in water, stayed at 5°C, and precipitated. |
| 32 | 180 | 600 | 30 | / | / | / | / | Soluble in water, stayed at 5°C, and precipitated |
| 33 | 45 | 400 | 7.5 | / | / | / | / | Soluble in water, stayed at 5°C, and no precipitation. |
| 34 | 90 | 600 | 15 | / | / | / | / | Soluble in water, stayed at 5°C, and precipitated |
| 35 | 90 | 900 | 15 | / | / | / | / | Soluble in water, stayed at 5°C, and no precipitation. |
| 36 | 100 | 400 | 10 | / | / | / | / | Soluble in pH6.8 phosphate medium |
| 37 | 100 | 500 | 10 | / | / | / | / | Soluble in pH6.8 phosphate medium |
| 38 | 100 | 600 | 10 | / | / | / | / | Soluble in pH6.8 phosphate medium |
| 39 | 53 | 280 | / | 9 | / | 1 | / | Failing to re-dissolve to be clear in pH6.8 phosphate medium |
| 40 | 53 | 280 | / | / | 10 | / | / | Soluble in pH6.8 phosphate medium |
| 41 | 53 | 280 | / | / | / | 3 | 7 | Soluble in pH6.8 phosphate medium |

| | | | | | | | | |
|---|---|---|---|---|---|---|---|---|
| Note: The active compounds in Examples 39 to 41 were fed in pure form | | | | | | | | |

After rotary evaporation, HP-β-CD inclusion complexes of active compounds were obtained in Examples 31 to 41. The HP-β-CD inclusion complexes of Examples 33, 35, 36, 37, 38, 40, and 41 were dissolved into clear and transparent aqueous solutions, without precipitation.

The eye drops of Examples 36, 37, and 38 were sterilized at 121°C for 15 minutes. The eye drops of Examples 36 and 37 became turbid, while the eye drops of Example 38 remained clear after sterilization.

The eye drops prepared in Examples 40 and 41 were sterilized at 121°C for 15 minutes, and the main components and maximum single impurity results are shown in Table 4. The main components of Example 40 and Example 41 decreased by 0.62% and 0.61% before and after sterilization, respectively; and the maximum single impurity increased by 1.61% and 1.35% before and after sterilization, respectively.

**Table 4**

| Examples | 121°C 15min sterilization | Peak area ratio | |
|---|---|---|---|
| | | Main component (%) | Maximum single impurity (%) |
| 40 | Before sterilization | 98.68 | 0.40 |
| | After sterilization | 98.06 | 2.01 |
| 41 | Before sterilization | 96.54 | 0.62 |
| | After sterilization | 95.93 | 1.97 |

### Examples 42-45

According to Table 5, 10 mL of isopropanol was taken and placed in a 50 mL beaker, stirred magnetically, added with the active compound, and stirred until dissolved; added with 4.00 g of HP-β-CD, stirred well, then added with 10 mL of isopropanol and sonicate for 12 minutes to obtain a clear solution. In Example 42, firstly, the solution was evaporated by rotation firstly at room temperature until it turned from clear state to white and solid precipitated, then transferred into a 50°C water bath, adjusted to vacuum of 0.085 MPa, resulting in boiling, and subjected to rotary evaporation for 1h, with the inclusion compound being scraped out. In example 43, the active compound was placed directly in a 50°C water bath, and the vacuum degree was adjusted to 0.085 MPa. Rotary evaporation was performed for 1 h, and the inclusion compound was scraped out. The stability results before and after rotary evaporation in Examples 42 and 43 are shown in Table 6. In Examples 44 and 45, the stability of the rotary evaporation solution was investigated at 50°C. The results are shown in Table 7.

**Table 5**

| Examples | Compound of Formula (IX) (g, anhydrous) | HP-β-CD (g) | Isopropanol (mL) | Insulation time at 50°C | rotary evaporation at room temperature | Evaporation time at 50°C |
|---|---|---|---|---|---|---|
| 42 | 0.5 | 4.00 | 20 | / | From clear to white, with solid precipitation | 1 h |
| 43 | 0.5 | 4.00 | 20 | / | / | 1 h |
| 44 | 0.5 | 4.00 | 20 | 1 h | / | / |
| 45 | 0.5 | 4.00 | 20 | 2 h | / | / |

**Table 6**

| Examples | Samples | Relevant substances (%) |
|---|---|---|
| | Control substance of raw materials | 0.97 |
| 42 | Solution before rotary evaporation | 0.97 |
| | Inclusion complex after rotary evaporation | 0.99 |
| 43 | Solution before rotary evaporation | 0.96 |
| | Inclusion complex after rotary evaporation | 0.97 |

**Table 7**

| Examples | Samples | Relevant substances (%) |
|---|---|---|
| | Control substance of raw materials | 0.77 |
| 44 | Insulation for 1 hour at 50°C | 0.82 |
| 45 | Insulation for 2 hours at 50°C | 0.83 |

The experimental results show that the relevant substances of the active compound formula (IX) remained basically unchanged before and after rotary evaporation in Examples 42 and 43, and the rotary evaporation process essentially does not affect the stability of the active compound. Before rotary evaporation, the solutions of Example 44 and Example 45 were kept at 50°C within 2 hours, and the relevant substances of the active compound remained basically unchanged.

### Examples 46-47

HP-β-CD and active compound of formula (IX) were placed in a beaker according to the ratio in Table 8, added with organic solvent, stirred to dissolve, and slowly added with purified water to obtain a clear solution. The solution was freeze dried to investigate the effect of different solvents on the intermediate state and reconstitution after freeze-drying.

**Table 8**

| Examples | Compound of Formula (IX) (mg, anhydrous) | HP-β-CD (mg) | 20% isopropanol (mL) | 20% tert-butanol (mL) |
|---|---|---|---|---|
| 46 | 100 | 560 | 50 | / |
| 47 | 100 | 560 | / | 50 |

The experimental results showed that, the intermediate of Example 46 is a white, loose, porous solid with good morphology. The intermediate of Example 47 is a white porous solid with loose pores, with solid powder splashing. The solutions of Example 46 and Example 47 were slightly turbid after reconstitution.

### Examples 48-51

In Example 48, Example 49, and Example 50, according to the prescription in Table 9, the active compound of formula (IX), HP-β-CD, butylated hydroxyanisole (BHA) and vitamin E (VE) were placed in a beaker, added with isopropanol, stirred well, slowly added with a certain amount of purified water to obtain a clear solution, and freeze dried. Example 51 used only quantitatively purified water to disperse the active compound before freeze-drying. The influence of different excipients on impurities under freeze-drying process was investigated. The main component and impurity content data of Examples 48-51 are shown in Table 10.

**Table 9**

| Examples | Compound of Formula (IX) (mg, anhydrous) | HP-β-CD (g) | BHA (mg) | VE (mg) | 20% isopropanol (mL) | Purified water (mL) |
|---|---|---|---|---|---|---|
| 48 | 200 | 1.12 | / | / | 20 | / |
| 49 | 200 | 1.12 | 160 | / | 20 | / |
| 50 | 200 | 1.12 | / | 160 | 20 | / |
| 51 | 200 | / | / | / | / | 20 |

**Table 10**

| Examples | 0 h | | 5 days at 60°C | |
|---|---|---|---|---|
| | Main component (%) | Maximum single impurity (%) | Main component (%) | Maximum single impurity (%) |
| 48 | 98.7 | 0.48 | 98.7 | 0.42 |
| 49 | 96.8 | 2.03 | 95.2 | 1.89 |
| 50 | 98.9 | 0.49 | 97.8 | 0.96 |
| 51 | 98.7 | 0.48 | 99.0 | 0.41 |

The results showed that there were no significant changes in the relevant substances of Example 48 and Example 51, and the addition of HP-β-CD did not affect the stability of the active compound.

Compared with Example 48, Example 49, with the addition of antioxidant BHA, achieved a maximum single impurity of 2.03% at 0 h. After a 5-day stability test at 60°C, it was found that, the content of the main components in Examples 49 and 50 showed different degrees of reduction compared to Example 48. Adding antioxidants BHA or VE is not conducive to the stability of active compound eye drops.

### Examples 52-63

According to the prescription ratio in Table 11, the active compound of formula (IX) was weighed, and dispersed in isopropanol with stirring until the solution was clear. HP-β-CD was slowly added under stirring, until the solution was clear. Purified water in the prescribed ratio was slowly added to the isopropanol solution under stirring, and stirred for 1 hour to obtain a clear solution. The above solution was freeze-dried in a vacuum freeze dryer to obtain a loose white freeze-dried powder. The freeze-drying curve is shown in Table 12. Freeze-dried powder of the inclusion compound was dissolved in a re-dissolving medium, and successively filtered through 0.45 µm and 0.22 µm mixed filter membrane to obtain active compound eye drops, and filled in a sample packaging of 2 mL per bottle, to obtain eye drops.

For multi-dose packaged eye drops, the addition of antimicrobial preservatives is needed to avoid microbial contamination during use. The effects of two antimicrobial preservatives, benzalkonium chloride and chlorhexidine acetate, on prescription stability were investigated through Examples 52, 55, and 56. The preliminary stability of eye drops from Example 52, Example 55, and Example 56 was investigated by placing them in the dark at 25°C and 40°C for 5 days. The results are shown in Table 13. Judging from the number and level of impurity growth, compared with Example 57, Example 56 has better short-term stability, and the antimicrobial preservative chlorhexidine acetate is more suitable for active compound eye drops.

Examples 53 and 54 were packaged in low-density polyethylene (PE) pharmaceutical eye drops bottles, brown polyester (PET) pharmaceutical eye drops bottles and ampoules, respectively. They were placed in the dark at 25°C to investigate the preliminary compatibility of the packaging materials. The stability data are shown in Table 14. Example 52 of isopropanol freeze-drying process production showed crystallization after long-term storage. Examples 53 and 54 with different packaging systems were left at 25°C for 15 days with no crystallization and no significant change in impurities in the eye drops.

When re-dissolving the products from Examples 57-60, dilute hydrochloric acid and dilute sodium hydroxide were used to adjust the pH value and investigate the appropriate acidity and alkalinity of the active compound of formula (IX) compound eye drops. The stability results of Examples 57-60 at 60°C for 5 days are shown in Table 15. The results show that the stability of eye drops under weakly alkaline conditions is better than that under acidic conditions.

The stability data of Examples 61-63 are shown in Table 16. Compared with Example 61, there was no significant change in the relevant substances of Example 62, and the addition of the antimicrobial preservative chlorhexidine acetate did not affect the stability of the active compound formula (IX) eye drops. Compared with Example 62, there was no significant change in the relevant substances in Example 63, and the increase in the amount of HP-β-CD added as a solubilizing agent did not affect the stability of the active compound.

**Table 11**

| Exam ples | Comp ound of Formu la (IX) (mg, anhydr ous) | HP-β-CD (g) | Isopro panol (mL) | Sodiu m dihydr ogen phosp hate (mg) | Disod ium hydro gen phosp hate (mg ) | Benzalk onium Chloride (mg) | Chlorhe xidine acetate (mg) | Sodi um chlo ride (m g) | Purif ied wate r (m L) | pH | Packaging material |
|---|---|---|---|---|---|---|---|---|---|---|---|
| | | | Purifie d water (mL) | | | | | | | | |
| 52 | 250 | 1.40 (2.8 %) | 4.50 | 200 | 235.5 | / | / | 250 | 50 | / | PET |
| | | | 18.00 | | | | | | | | |
| 53 | 250 | 1.75 (3.5 %) | 4.50 | 200 | 235.5 | / | / | 250 | 50 | / | PET/PE/a mpoule |
| | | | 18.00 | | | | | | | | |
| 54 | 250 | 2.00 (4.0 %) | 4.50 | 200 | 235.5 | / | / | 250 | 50 | / | PET/PE/a mpoule |
| | | | 18.00 | | | | | | | | |
| 55 | 250 | 1.40 (2.8 %) | 4.50 | 200 | 235.5 | 5.0 | / | 250 | 50 | / | PET |
| | | | 18.00 | | | | | | | | |
| 56 | 250 | 1.40 (2.8 %) | 4.50 | 200 | 235.5 | / | 5.0 | 250 | 50 | / | PET |
| | | | 18.00 | | | | | | | | |
| 57 | 125 | 0.70 (2.8 %) | 4.25 | 100 | 117.8 | / | / | / | 25 | 2.0 30 | ampoule |
| | | | 12.75 | | | | | | | | |
| 58 | 125 | 0.70 (2.8 %) | 4.25 | 100 | 117.8 | / | / | / | 25 | 5.0 46 | ampoule |
| | | | 12.75 | | | | | | | | |
| 59 | 125 | 0.70 (2.8 %) | 4.25 | 100 | 117.8 | / | / | / | 25 | 7.1 54 | ampoule |
| | | | 12.75 | | | | | | | | |
| 60 | 125 | 0.70 (2.8 %) | 4.25 | 100 | 117.8 | / | / | / | 25 | 9.0 27 | ampoule |
| | | | 12.75 | | | | | | | | |
| 61 | 1375 | 9.75 (3.5 %) | 47.15 | 1100 | 1295. 0 | / | / | 715 | 275 | / | PET |
| | | | 141.40 | | | | | | | | |
| 62 | 1375 | 9.75 (3.5 %) | 47.15 | 1100 | 1295. 0 | / | 27.5 | 715 | 275 | / | PET |
| | | | 141.40 | | | | | | | | |
| 63 | 1375 | 11.00 (4.0 %) | 47.15 | 1100 | 1295. 0 | / | 27.5 | 715 | 275 | / | PET |
| | | | 141.40 | | | | | | | | |

**Table 12**

| Phase | Temperature (°C) | Phase time (min) | Time (min) |
|---|---|---|---|
| First phase | -40 | 0 | 0 |
| Second phase | -30 | 60 | 60 |
| Third phase | -30 | 600 | 720 |
| Forth phase | -15 | 180 | 900 |
| Fifth phase | -15 | 120 | 1020 |
| Sixth phase | -5 | 120 | 1140 |
| Seventh phase | -5 | 360 | 1500 |
| Eighth phase | 15 | 120 | 1620 |
| Ninth phase | 15 | 120 | 2820 |

**Table 13**

| Retention time (min) | Relative retention time | Compound of Formula (IX) | Example 52 | | Example 55 | | Example 56 | |
|---|---|---|---|---|---|---|---|---|
| | | 0 h | 25°C 5 days | 40°C 5 days | 25°C 5 days | 40°C 5 days | 25°C 5 days | 40°C 5 days |
| 19.6 | 1.00 | 93.14 | 93.03 | 93.12 | 92.97 | 92.80 | 93.13 | 92.81 |
| 20.2 | 1.03 | 0.16 | 0.16 | 0.15 | 0.16 | 0.16 | 0.16 | 0.16 |
| 21.1 | 1.08 | 0.39 | 0.46 | 0.26 | 0.43 | 0.27 | 0.37 | 0.27 |
| 21.5 | 1.10 | 0.41 | 0.39 | 0.38 | 0.40 | 0.38 | 0.40 | 0.39 |
| 23.8 | 1.21 | 0.11 | / | 0.09 | / | 0.08 | 0.08 | 0.08 |
| 24.0 | 1.22 | 2.41 | 2.57 | 2.31 | 2.67 | 2.41 | 2.45 | 2.46 |
| 24.5 | 1.25 | 0.50 | 0.57 | 0.52 | 0.52 | 0.54 | 0.49 | 0.49 |
| 24.7 | 1.26 | 0.25 | 0.33 | 0.30 | 0.27 | 0.30 | 0.27 | 0.26 |
| 24.8 | 1.27 | 0.12 | 0.14 | 0.18 | 0.12 | 0.18 | 0.12 | 0.16 |
| 25.6 | 1.31 | 0.09 | / | / | / | / | / | 0.09 |
| 26.0 | 1.33 | 0.15 | 0.15 | 0.14 | 0.19 | 0.17 | 0.13 | 0.14 |
| 26.2 | 1.34 | 2.15 | 2.20 | 2.06 | 2.27 | 2.21 | 2.16 | 2.19 |
| 26.7 | 1.36 | / | / | 0.25 | / | 0.25 | 0.11 | 0.26 |
| 27.0 | 1.38 | / | / | 0.11 | / | 0.11 | / | 0.11 |
| 28.3 | 1.44 | 0.13 | / | 0.13 | / | 0.13 | 0.13 | 0.14 |

**Table 14**

| RRT/Nam e | Compoun d of Formula (IX) | Blank Matri x | Example | | | | | | | | |
|---|---|---|---|---|---|---|---|---|---|---|---|
| | | | 52 | | | 53 | | | 54 | | |
| | | | 25°C 20 days PET bottl e | 25°C 30 days PET bottl e | 25°C5 0 days PET bottle | 25°C 15 days PET bottl e | 25°C 15 days PE bottl e | 25°C 15 days ampoul e | 25°C 15 days PET bottl e | 25°C 15 days PE bottl e | 25°C 15 days ampoul e |
| 0.44 | 0.06 | N.D. | 0.08 | 0.08 | 0.07 | 0.06 | 0.06 | 0.06 | 0.06 | 0.06 | 0.06 |
| 1.17 | 0.05 | N.D. | 0.03 | 0.03 | N.D. | 0.02 | 0.02 | 0.02 | 0.02 | 0.02 | 0.02 |
| 1.29 | 0.20 | N.D. | 0.25 | 0.24 | 0.22 | 0.19 | 0.19 | 0.19 | 0.19 | 0.19 | 0.19 |
| 1.34 | 0.10 | N.D. | 0.11 | 0.10 | 0.11 | 0.12 | 0.10 | 0.10 | 0.10 | 0.10 | 0.10 |
| 1.51 | N.D. | N.D. | N.D. | N.D. | N.D. | 0.03 | 0.03 | 0.03 | 0.03 | 0.03 | 0.03 |
| 1.55 | 0.07 | N.D. | 0.11 | 0.10 | 0.09 | 0.08 | 0.08 | 0.08 | 0.08 | 0.08 | 0.08 |
| 1.59 | 0.02 | N.D. | N.D. | N.D. | N.D. | 0.02 | N.D. | N.D. | 0.02 | N.D. | N.D. |
| 1.66 | 0.04 | N.D. | 0.06 | 0.06 | 0.05 | 0.04 | 0.04 | 0.04 | 0.04 | 0.04 | 0.04 |
| 1.75 | 0.04 | N.D. | 0.37 | 0.49 | 0.66 | 0.05 | 0.04 | 0.04 | 0.05 | 0.05 | 0.05 |
| 1.81 | 0.03 | N.D. | N.D. | N.D. | N.D. | N.D. | N.D. | N.D. | N.D. | N.D. | N.D. |
| 1.84 | 0.25 | N.D. | 0.14 | 0.14 | 0.09 | 0.10 | 0.10 | 0.09 | 0.09 | 0.09 | 0.09 |
| 1.89 | 0.04 | N.D. | 0.79 | 1.04 | 1.44 | 0.02 | 0.02 | N.D. | N.D. | N.D. | N.D. |
| 1.97 | 0.03 | N.D. | N.D. | N.D. | N.D. | N.D. | N.D. | N.D. | N.D. | N.D. | N.D. |
| 2.10 | 0.09 | N.D. | 0.06 | 0.06 | 0.05 | 0.05 | 0.05 | 0.04 | 0.05 | 0.04 | 0.04 |
| 2.19 | 0.21 | N.D. | 0.12 | 0.11 | 0.09 | 0.10 | 0.09 | 0.10 | 0.10 | 0.09 | 0.09 |
| 2.23 | 0.02 | N.D. | 0.09 | 0.13 | 0.17 | 0.13 | 0.14 | 0.14 | 0.12 | 0.12 | 0.12 |
| 2.27 | 0.03 | N.D. | N.D. | N.D. | 0.03 | 0.05 | 0.05 | 0.05 | 0.04 | 0.04 | 0.04 |
| Total impurity | 1.03 | N.D. | 2.18 | 2.55 | 3.04 | 0.93 | 0.86 | 0.81 | 0.84 | 0.78 | 0.78 |

**Table 15**

| Example | Relevant substance at 0 h | | Relevant substance at 60°C on 5 days | |
|---|---|---|---|---|
| | Main component (%) | Maximum single impurity (%) | Main component (%) | Maximum single impurity (%) |
| 57 | 92.34 | 2.61 | 76.04 | 13.55 |
| 58 | 92.52 | 2.45 | 89.69 | 2.74 |
| 59 | 92.97 | 2.30 | 92.69 | 2.21 |
| 60 | 93.38 | 2.12 | 93.11 | 2.07 |

**Table 16**

| Ex am ple | 0 h | | 25°C 10 days | | 25°C 30 days | | 40°C 10 days | | 40°C 20 days | | 40°C 30 days | |
|---|---|---|---|---|---|---|---|---|---|---|---|---|
| | Main compo nent (%) | Maxi mum single impur ity (%) | Main compo nent (%) | Maxi mum single impur ity (%) | Main compo nent (%) | Maxi mum single impur ity (%) | Main compo nent (%) | Maxi mum single impur ity (%) | Main compo nent (%) | Maxi mum single impur ity (%) | Main compo nent (%) | Maxi mum single impur ity (%) |
| 61 | 99.23 | 0.26 | 98.90 | 0.23 | 97.20 | 0.21 | 98.87 | 0.21 | 98.28 | 0.26 | 98.57 | 0.25 |
| 62 | 99.22 | 0.26 | 99.07 | 0.24 | 99.04 | 0.21 | 98.86 | 0.21 | 98.71 | 0.27 | 98.62 | 0.27 |
| 63 | 99.18 | 0.25 | 99.12 | 0.23 | 99.07 | 0.20 | 98.80 | 0.23 | 98.66 | 0.22 | 98.73 | 0.22 |

### Examples 64~67

According to the prescription ratio in Table 17, 30 g of purified water was heated to 80°C, and added with hydroxypropyl betadex (HP-β-CD) and sulfobutylether-β-cyclodextrin (SBE-β-CD) at small amounts by batches under stirring for dissolving. The prescription amounts of active compound of formula (IX) were weighed separately, and added in small amounts by batches to the cyclodextrin solution under stirring. The active compounds in Examples 64, 66, and 67 can be completely dissolved within 2 hours. Purified water was added to a volume of 60 mL and stirred evenly in Examples 64-66. The above solution was encapsulated into medium borosilicate glass ampoules, 4 mL per vial, for a total of 10 vials, obtaining eye drops ready for use.

Products obtained in Example 64 and Example 66 were placed in an 80°C water bath and heated away from light. Two ampoules were taken out at 0.5, 1, 2, 4, and 6 hours, respectively, and the samples were sent to determine the content and relevant substances. The results are shown in Table 18.

Example 64 showed an increase of 0.03%, 0.10%, and 0.21% of relevant substances after 0.5, 1, and 2 hours at 80°C, respectively; Example 66 showed an increase of 0.30%, 0.47%, and 0.57% of relevant substances after 0.5, 1, and 2 hours at 80°C. Compared with Example 64, Example 66 containing SBE-β-CD is less thermal stable.

**Table 17**

| Example s | Compound of Formula (IX) (g) | HP-β-CD (g) | SBE-β-CD (g) | Purified water (mL) | Adding purified water to (mL) |
|---|---|---|---|---|---|
| 64 | 3.66 | 24 | / | 30 | 60 |
| 65 | 3.00 | / | 24 | 30 | 60 |
| 66 | 2.55 | / | 24 | 30 | 60 |
| 67 | 1.02 | / | 24 | 30 | 60 |

**Table 18**

| Sample | | Example 64 | Example 66 |
|---|---|---|---|
| Content (mg/mL) | | 59.45 | 40.30 |
| Relev ant substa nces (%) | Control Substance | 0.56 | 0.59 |
| | 80°C; 0.5h | 0.59 (↑0.03) | 0.89 (↑0.30) |
| | 80°C; 1h | 0.66 (↑0.10) | 1.06 (↑0.47) |
| | 80°C; 2h | 0.77 (↑0.21) | 1.16 (↑0.57) |
| | 80°C; 4h | 0.97 (↑0.41) | 1.26 (↑0.67) |
| | 80°C; 6h | 1.17 (↑0.61) | 1.45 (↑0.86) |

### Examples 68-91

Examples 68-87 were prepared according to the prescription ratio in Table 19. The active compound of formula (IX) and HP-β-CD were weighed and placed in a 10 mL vial, and added with purified water according to the prescription ratio under stirring or shaking at different water bath temperatures until dissolution. The dissolution of the active compound under different concentration process conditions was compared. Example 79 was diluted by 1 and 5 times to obtain Example 88 and Example 89. Examples 90 and 91 were obtained by diluting Example 80 by 1 and 5 times.

Under the concentration conditions listed in Table 19, the active compound of formula (IX) were completely dissolved in a short period of time in Examples 69, 70, 75-83, 85, and 86. Under the concentration process conditions, the dosage of HP-β-CD, concentration of HP-β-CD, and concentration process temperature have a significant impact on the dissolution process of active compounds.

**Table 19**

| Exa mple s | Compound of Formula (IX) (%, anhydrous) | HP-β-CD (%) | Concent ration temperat ure (°C) | HP-β-CD concentratio n (%) | Operating method | Dissolvi ng time (min) | Test results |
|---|---|---|---|---|---|---|---|
| 68 | 0.5 | 4 | 30 | 9 | 200 rpm shaking | 120 | Incomplete dissolution |
| 69 | 0.5 | 8 | 30 | 20 | 200 rpm shaking | 10 | Complete dissolution |
| 70 | 0.5 | 7.5 | 30 | 20 | Ultrasound | 10 | Complete dissolution |
| 71 | 0.5 | 6 | 30 | 20 | Ultrasound | 60 | Incomplete dissolution |
| 72 | 0.5 | 6 | 30 | 15 | Ultrasound | 60 | Incomplete dissolution |
| 73 | 0.5 | 4 | 25 | 16 | Stirring | 60 | Incomplete dissolution |
| 74 | 0.5 | 4 | 25 | 28 | Stirring | 60 | Incomplete dissolution |
| 75 | 0.5 | 6 | 40 | 23 | Stirring | 60 | Complete dissolution |
| 76 | 0.5 | 7 | 40 | 25 | Stirring | 45 | Complete dissolution |
| 77 | 0.5 | 6 | 40 | 23 | Stirring | 60 | Complete dissolution |
| 78 | 0.5 | 8 | 25 | 43 | Stirring | 30 | Complete dissolution |
| 79 | 0.5 | 6 | 40 | 43 | Stirring | 60 | Complete dissolution |
| 80 | 0.5 | 5 | 50 | 42 | Stirring | 60 | Complete dissolution |
| 81 | 0.5 | 3.5 | 80 | 42 | Stirring | 30 | Complete dissolution |
| 82 | 0.5 | 3.5 | 70 | 42 | Stirring | 60 | Complete dissolution |
| 83 | 0.5 | 3.5 | 70 | 44 | Stirring | 30 | Complete dissolution |
| 84 | 0.5 | 3.5 | 70 | 29 | Stirring | 200 | Incomplete dissolution |
| 85 | 0.5 | 3.5 | 75 | 35 | Stirring | 30 | Complete dissolution |
| 86 | 0.5 | 3.5 | 75 | 44 | Stirring | 30 | Complete dissolution |
| 87 | 0.5 | 3.0 | 75 | 40 | Stirring | 60 | Incomplete dissolution |

The stability of the aqueous solution of the active compound inclusion complexes listed in Table 20 and the photostability of Example 80 were investigated. The results are shown in Tables 21, 22, and 23.

The experimental results show that the content of active compounds remained stable after 10 days at 60°C and 20 days at 25°C, 30°C, and 40°C, indicating that the thermal stability of cyclodextrin aqueous solutions containing active compounds in Examples 67, 78-80, and 88-91 was still acceptable. In Example 80, a yellow precipitate appeared after 5 days of investigation under the influencing factor test light conditions, indicating that the cyclodextrin aqueous solution containing the active compound is unstable to strong light.

**Table 20**

| Example | Specification (mg/mL) | Cyclodextrin/Dosage |
|---|---|---|
| 67 | 5 | SBE-β-CD/12.5% |
| 78 | 5 | HP-β-CD/8% |
| 79 | 5 | HP-β-CD/6% |
| 80 | 5 | HP-β-CD/5% |
| 88 | 2.5 | HP-β-CD/3% |
| 89 | 1 | HP-β-CD/1.2% |
| 90 | 2.5 | HP-β-CD/2.5% |
| 91 | 1 | HP-β-CD/1% |

**Table 21**

| Examples | Content (mg/mL) | | | | | | | |
|---|---|---|---|---|---|---|---|---|
| | 0h | 60°C 3 days | 60°C 5 days | 60°C 10 days | 40°C 10 days | 40°C 20 days | 30°C 20 days | 25°C 20 days |
| 67 | 4.81 | 4.82 | 4.89 | 4.91 | / | / | / | / |
| 78 | 4.79 | 4.60 | 4.69 | 4.67 | 4.77 | / | / | / |
| 79 | 5.00 | 4.87 | 4.87 | 4.91 | 4.99 | 4.90 | 4.89 | 4.85 |
| 80 | 4.88 | 4.92 | 4.90 | 4.86 | 4.89 | 4.86 | 4.89 | 4.89 |
| 88 | 2.47 | 2.37 | 2.46 | 2.40 | 2.46 | 2.38 | 2.42 | 2.41 |
| 89 | 0.99 | 0.96 | 0.98 | 0.98 | 0.98 | 0.96 | 0.99 | 0.98 |
| 90 | 2.42 | 2.35 | 2.44 | 2.48 | 2.44 | 2.44 | 2.44 | 2.43 |
| 91 | 0.96 | 0.96 | 0.96 | 0.98 | 0.98 | 0.99 | 0.98 | 0.98 |

**Table 22**

| Examples | Relevant substances (%) | 60°C 3 days | 60°C 5 days | 60°C 10 days | 40°C 20 days | 30°C 20 days | 25°C 20 days |
|---|---|---|---|---|---|---|---|
| 67 | Maximum single impurity | 0.07 | 0.07 | 0.10 | / | / | / |
| | Total impurities | 0.68 | 0.65 | 0.78 | / | / | / |
| 78 | Maximum single impurity | 0.11 | 0.11 | 0.12 | / | / | / |
| | Total impurities | 0.90 | 0.89 | 1.01 | / | / | / |
| 79 | Maximum single impurity | 0.07 | 0.07 | 0.07 | 0.07 | 0.07 | 0.07 |
| | Total impurities | 0.68 | 0.74 | 0.77 | 0.71 | 0.65 | 0.61 |
| 80 | Maximum single impurity | 0.07 | 0.08 | 0.08 | 0.07 | 0.07 | 0.07 |
| | Total impurities | 0.71 | 0.71 | 0.80 | 0.70 | 0.62 | 0.56 |
| 88 | Maximum single impurity | 0.07 | 0.07 | 0.10 | 0.07 | 0.07 | 0.07 |
| | Total impurities | 0.66 | 0.66 | 0.78 | 0.68 | 0.65 | 0.62 |
| 89 | Maximum single impurity | 0.08 | 0.07 | 0.07 | 0.07 | 0.07 | 0.07 |
| | Total impurities | 0.75 | 0.67 | 0.70 | 0.60 | 0.59 | 0.58 |
| 90 | Maximum single impurity | 0.07 | 0.07 | 0.07 | 0.07 | 0.07 | 0.07 |
| | Total impurities | 0.67 | 0.69 | 0.75 | 0.70 | 0.63 | 0.61 |
| 91 | Maximum single impurity | 0.07 | 0.07 | 0.09 | 0.07 | 0.07 | 0.07 |
| | Total impurities | 0.66 | 0.64 | 0.76 | 0.64 | 0.63 | 0.57 |

**Table 23**

| Examples | Content | Relevant substances (%) | |
|---|---|---|---|
| | | Total impurities | Maximum single impurity |
| Example 80 (illumination for 5 days) | 4.34 mg/mL (88.93%) | 3.15 | 0.629 |

### Example 92

Example 92 was prepared according to the prescription ratio in Table 24. A certain amount of purified water was added to a beaker, heated in a water bath to 70°C, added with HP-β-CD and stir to dissolve, then added with the active compound of formula (IX), and stirred in a water bath at 70°C for 60 minutes until completely dissolved. The solution was transferred into a 100 mL volumetric flask. The beaker was rinsed several times with a small amount of purified water, with the rinsing water being transferred into the volumetric flask, added with chlorhexidine acetate, shaken for dissolving, added with dibasic sodium phosphate, monobasic sodium phosphate monohydrate, and sodium chloride in sequence, shaken for dissolving, and added with purified water to make up to 100 mL. The solution was filtered through 0.45 µm and 0.22 µm mixed membranes in sequence, and the filtrate was filled in 5 mL medium borosilicate glass ampoules by 5 mL per vial to obtain eye drops for use.

Example 92 eye drops were sterilized at 115°C for 30 minutes, 121°C for 15 minutes, and 121°C for 30 minutes, respectively. The property, pH, and relevant substances of the samples were tested. The results are shown in Table 25. Example 92 was tested at 60°C, and samples were taken at 0, 5, and 10 days to detect their property, content, and relevant substances. The test results are shown in Table 26.

**Table 24**

| Example | Example 92 |
|---|---|
| Compound of formula (IX) (anhydrous) | 0.50 g |
| HP-β-CD (solubilizing agent) | 3.50 g |
| Dibasic sodium phosphate (pH modifier) | 0.47 g |
| Monobasic sodium phosphate monohydrate (pH modifier) | 0.40 g |
| Sodium chloride (osmotic pressure regulator) | 0.34 g |
| Chlorhexidine acetate (antimicrobial preservative) | 0.01 g |
| Adding injection water to | 100 mL |

**Table 25**

| Sample | Property | pH | Relevant substances (%) | |
|---|---|---|---|---|
| | | | Maximum single impurity | Total impurities |
| Before sterilization | Colorless clear liquid | 6.80 | 0.076 | 0.644 |
| Sterilizing at 115°C for 30min | Colorless clear liquid | 6.80 | 1.206 | 2.151 |
| Sterilizing at 121°C for 15min | Colorless clear liquid | 6.80 | 1.342 | 2.331 |
| Sterilizing at 121°C for 30min | Colorless clear liquid | 6.80 | 1.852 | 2.839 |

**Table 26**

| Sample | Property | pH | Relevant substances (%) | | Content (mg/mL) |
|---|---|---|---|---|---|
| | | | Maximum single impurity | Total impuri ties | |
| 0 day | Colorless clear liquid | 6.80 | 0.076 | 0.644 | 4.84 |
| 5 days at 60°C | Colorless clear liquid | 6.80 | 0.354 | 1.275 | 4.80 |
| 10 days at 60°C | Colorless clear liquid | 6.79 | 0.700 | 1.672 | 4.80 |

The results showed that, after sterilization under different conditions, the relevant substances in Example 92 increased significantly. The total impurities in the samples sterilized at 115°C for 30 minutes increased by 1.507%, and the total impurities in the samples sterilized at 121°C for 15 minutes and 30 minutes increased by 1.687% and 2.195%, respectively. Example 92 had poor thermal stability, and cannot tolerate moist heat sterilization. After being tested at 60 °C for 5 and 10 days, the relevant substances in Example 92 increased, while other indicators remained stable.

### Example 93

Example 93 was prepared according to the prescription ratio in Table 27. 21.86 g of purified water was added to a 100 mL beaker, heated to 60°C, added with HP-β-CD under stirring for dissolving, then added with the active compound of formula (IX), heated to 70°C and stirred for 60 minutes until completely dissolved. The solution was transferred into a 500 mL volumetric flask, and the beaker was rinsed several times with about 200 g of purified water. The rinsing water was transferred into the volumetric flask, added with chlorhexidine acetate, shaken to dissolve, then added with dibasic sodium phosphate, monobasic sodium phosphate monohydrate, and sodium chloride in sequence, shaken to dissolve, added with purified water to make up to 500 mL, and shaken well. The density of the solution was calculated to be 1.0166 g/mL based on the mass to volume ratio. The solution was filled into 5 mL borosilicate glass ampoules by 5 mL/vial to obtain eye drops.

Example 93 was placed about 1.5 meters under a 40 watt indoor fluorescent lamp, and samples were taken at 0, 2, 4, and 6 hours to test their properties, pH, and relevant substances. The test results are shown in Table 28. The results showed that Example 93 remained stable after being placed about 1.5 meters under the indoor fluorescent lamps for 6 hours, and both liquid formulating and filling during production could be carried out under normal indoor lighting.

**Table 27**

| Example | Example 93 |
|---|---|
| Compound of formula (IX) (anhydrous) | 2.50 g |
| HP-β-CD (solubilizing agent) | 17.50 g |
| Dibasic sodium phosphate (pH modifier) | 2.35 g |
| monobasic sodium phosphate monohydrate(pH modifier) | 2.00 g |
| Sodium chloride (osmotic pressure regulator) | 1.70 g |
| Chlorhexidine acetate (antimicrobial preservative) | 0.05 g |
| Adding injection water to | 500 mL |

**Table 28**

| Sample | Property | pH | Relevant substances (%) | |
|---|---|---|---|---|
| | | | Maximum single impurity | Total impurities |
| 0h | Colorless clear liquid | 6.78 | 0.051 | 0.409 |
| Indoor light exposure for 2 h | Colorless clear liquid | 6.78 | 0.085 | 0.403 |
| Indoor light exposure for 4h | Colorless clear liquid | 6.79 | 0.068 | 0.426 |
| Indoor light exposure for 6h | Colorless clear liquid | 6.78 | 0.068 | 0.384 |

### Examples 94-96

10.00 g of purified water was weighed and filled in a 50 mL beaker, heated in a water bath to 75°C, added with HP-β-CD according to the prescription in Table 29, stirred until dissolved, then added with the active compound formula (IX) under stirring until dissolved. Cyclodextrin solution containing active compounds was added to a 500 mL beaker containing 126 mL of purified water, and the 50 mL beaker was rinsed several times with purified water. The rinsing solution was transferred into the 500 mL beaker, added with chlorhexidine acetate, stirred until dissolved, then added with sodium chloride, monobasic sodium phosphate monohydrate, and dibasic sodium phosphate in sequence, stirred until dissolved, added with water to 250 mL, stirred well, and filtered through 0.45 µm and 0.22 µm mixed filter membranes to obtain Example 94.

The pH of Example 94 was 7.75. The solution of Example 94 was adjusted, with 1 mol/L HCl and 5 mol/L NaOH, respectively, in terms of the pH, to 6.96 to obtain Example 95; and to 8.54 to obtain Example 96. Eye drops of Example 94 were sterilized at 115°C for 30 minutes, 121°C for 15 minutes, and 121°C for 30 minutes, respectively. The property, pH, and relevant substances of the samples were tested. The results are shown in Table 30. The eye drops of Example 94, Example 95, and Example 96 were filled into 5 mL ampoules and placed at 60°C for examination. Samples were taken at 0, 5, and 10 days to test their property, content, and relevant substances. The test results are shown in Table 31.

The results showed that, the relevant substances in Example 94 increased significantly after sterilization under different conditions. Example 94 is not thermally stable and does not tolerate moist heat sterilization. The sample of Example 96 at pH 8.54 showed the best stability. In addition, considering that the reasonable pH range for eye drops is 6-8, the pH of Example 94 eye drops is more suitable.

**Table 29**

| Example | Example **94** |
|---|---|
| Compound of formula (IX) (anhydrous) | 1.250 g |
| HP-β-CD (solubilizing agent) | 8.750 g |
| Dibasic sodium phosphate (pH modifier) | 2.025 g |
| Monobasic sodium phosphate monohydrate (pH modifier) | 0.300 g |
| Sodium chloride (osmotic pressure regulator) | 0.675 g |
| Chlorhexidine acetate (antimicrobial preservative) | 0.025 g |
| Adding injection water to | 250 mL |

**Table 30**

| Example 94 | Property | pH | Relevant substances (%) |
|---|---|---|---|
| Before sterilization | Colorless clear liquid | 7.75 | 0.521 |
| Sterilizing at 115°C for 30 min | Colorless clear liquid | 7.75 | 1.031 |
| Sterilizing at 121°C for 15 min | Colorless clear liquid | 7.74 | 1.179 |
| Sterilizing at 121°C for 30 min | Colorless clear liquid | 7.75 | 1.425 |

**Table 31**

| Examples | Sample | Property | pH | Relevant substances (%) |
|---|---|---|---|---|
| 94 | 0 day | Colorless clear liquid | 7.75 | 0.521 |
| | 5 days at 60°C | Colorless clear liquid | 7.74 | 0.831 |
| | 10 days at 60°C | Colorless clear liquid | 7.75 | 1.494 |
| 95 | 0 day | Colorless clear liquid | 6.96 | 0.548 |
| | 5 days at 60°C | Colorless clear liquid | 6.96 | 1.201 |
| | 10 days at 60°C | Colorless clear liquid | 6.96 | 2.179 |
| 96 | 0 day | Colorless clear liquid | 8.54 | 0.523 |
| | 5 days at 60°C | Colorless clear liquid | 8.55 | 0.633 |
| | 10 days at 60°C | Colorless clear liquid | 8.55 | 1.149 |

### Examples 97-98

10.00 mL of purified water were weighed and placed in a 50 mL beaker, heated in a water bath to 75°C, added with HP-β-CD according to the prescription in Table 32, stirred until dissolved, and added with the active compound under stirring until dissolved. The cyclodextrin solution containing the active compound formula (IX) was added to a 500 mL beaker containing 126 mL of purified water, and the 50 mL beaker was rinsed several times with purified water. The rinsing solution was transferred into the 500 mL beaker, added with chlorhexidine acetate, stirred until dissolved, then added with sodium chloride, monobasic sodium phosphate monohydrate, and dibasic sodium phosphate in sequence, stirred until dissolved, added with water to 250 mL, stirred well, and filtered through 0.45 µm and 0.22 µm mixed filter membranes to obtain Example 97 and Example 98.

The osmotic pressure of Examples 94, 97, and 98 was measured. The results are shown in Table 33. The eye drops of Examples 94, 97, and 98 were in a state of near isotonic and slightly hypotonic.

**Table 32**

| Example | Example **97** | Example **98** |
|---|---|---|
| Compound of formula (IX) (anhydrous) | 0.625 g | 0.250 g |
| HP-β-CD (solubilizing agent) | 4.375 g | 1.750 g |
| Dibasic sodium phosphate (pH modifier) | 2.025 g | 2.025 g |
| Monobasic sodium phosphate monohydrate(pH modifier) | 0.300 g | 0.300 g |
| Sodium chloride (osmotic pressure regulator) | 0.900 g | 1.000 g |
| Chlorhexidine Acetate (Antimicrobial preservative) | 0.025 g | 0.025 g |
| Adding injection water to | 250 mL | 250 mL |

**Table 33**

| Example | Property | Osmotic Pressure Molar Concentration Ratio |
|---|---|---|
| 94 | Colorless clear liquid | 0.981 |
| 97 | Colorless clear liquid | 0.995 |
| 98 | Colorless clear liquid | 0.998 |

### Examples 99~102

According to the prescription ratio in Table 34, Examples 99-102 were prepared.

### Production process:

Concentrated solution preparation: adding the prescribed amount of injection water into a beaker, placing the beaker on a CNC heating type magnetic stirrer for heating to water temperature of 77°C± 2°C, and adding HP-β-CD in small amounts under stirring by batches.

After HP-β-CD was completely dissolved, stirring was kept and the prescribed amount of active compound was added for one time. The solution temperature was controlled at 77°C± 2°C and stirring was performed until the solution was completely clear.

Diluting concentrated solution to volume: weighing 7.5 kg of injection water at 20°C~25°C into a stainless steel bucket, starting the mixer to stir, adding the concentrated solution into the stainless steel bucket, rinsing the beaker of the concentrated solution for 4-6 times by using a small amount of injection water at 20°C~25°C, transferring all the rinsing solution to the stainless steel bucket and stirring evenly, adding the prescribed amount of sodium chloride, monobasic sodium phosphate monohydrate, and dibasic sodium phosphate in sequence, stirring for dissolving, dilute the solution to 15 L with injection water at 20°C~25°C, stirring evenly, and taking samples for intermediate properties, content, pH value, and osmotic pressure testing. The test results are shown in Table 35.

Filtering and filling: before production begins, conducting integrity test of the filter cartridge, flushing and conducting steam-in-place on the mixing tank, the Blow-Fill-Seal (BFS) machine, and their material conveying pipelines, maintaining a temperature of ≥ 121°C for 30 minutes for pure steam sterilization, commissioning the equipment, starting filtering and filling, and conducting integrity test on the filter cartridge after production was completed.

Punching: conducting leakage detection on all punched products, then inspecting each bottle and removing any non-conforming products.

Packaging: conducting light inspection on all qualified punched products to eliminate non-conforming products, performing pillow packaging on punched products that pass leakage detection and light inspection, testing the heat sealing effect of all packaged samples by using a vacuum constant temperature drying oven, taking samples from the heat sealed qualified finished products for inspection, and conducting tests on the property, content, pH value, and osmotic pressure of the finished products. The test results are shown in Table 35.

The results showed that, the prescription ratios and preparation processes of Examples 99 to 102 can produce eye drops and eye drop placebos with properties, contents, pH values, and osmotic pressures that comply with the 2020 Pharmacopoeia regulations.

**Table 34**

| Step | Material Name | Example 99 | | Example 100 | | Example 101 | | Example 102 | |
|---|---|---|---|---|---|---|---|---|---|
| | | % | g | % | g | % | g | % | g |
| Preparat ion of concentr ated solution | Compound of formula (III) | 0.10 | 15.0 | 0.25 | 37.5 | 0.50 | 75.0 | / | / |
| | HP-β-CD | 0.70 | 105.0 | 1.75 | 262.5 | 3.50 | 525.0 | 3.50 | 525.0 |
| | Injection water | / | 105.0 | / | 262.5 | / | 525.0 | / | 525.0 |
| Diluting of concentr ated solution | Dibasic sodium phosphate | 0.81 | 121.5 | 0.81 | 121.5 | 0.81 | 121.5 | 0.81 | 121.5 |
| | Monobasic sodium phosphate monohydrate | 0.12 | 18.0 | 0.12 | 18.0 | 0.12 | 18.0 | 0.12 | 18.0 |
| | Sodium chloride | 0.40 | 60.0 | 0.36 | 54.0 | 0.27 | 40.5 | 0.27 | 40.5 |
| | Injection water | To 15 L | | To 15 L | | To 15 L | | To 15 L | |

**Table 35**

| Example | Sample | Property | Indicated content (%) | pH | Osmotic pressure molar concentration ratio |
|---|---|---|---|---|---|
| 99 | Intermediate | Colorless clear liquid | 98.81 | 7.57 | 1.00 |
| | Finished product | Colorless clear liquid | 97.5 | 7.59 | 0.98 |
| 100 | Intermediate | Colorless clear liquid | 101.20 | 7.59 | 1.00 |
| | Finished product | Colorless clear liquid | 98.5 | 7.60 | 0.98 |
| 101 | Intermediate | Colorless clear liquid | 101.38 | 7.60 | 0.99 |
| | Finished product | Colorless clear liquid | 98.3 | 7.62 | 0.97 |
| 102 | Intermediate | Colorless clear liquid | / | 7.63 | 0.98 |
| | Finished product | Colorless clear liquid | / | 7.63 | 0.96 |

### Example 103

According to the prescription ratio in Table 36, sulfobutylether-β-cyclodextrin (SBE-β-CD) was weighed and dissolved in phosphate buffer solution. The prescripted amount of the compound of formula (IX) was dissolved in 0.15 mL of dimethyl sulfoxide, and then added to sulfobutylether-β-cyclodextrin (SBE-β-CD) phosphate buffer under stirring continuously to obtain a clear solution.

In Example 103, the amount of sulfobutylether-β-cyclodextrin used is high, and the fact that dimethyl sulfoxide has local toxicity and low systemic toxicity, making it unsuitable for use in eye drops. Compared with Examples 66 and 67, which have a simple composition of excipients and do not adding dimethyl sulfoxide, Example 103 has poor stability and higher safety risks.

**Table 36**

| Example | Compound of formula (IX) | SBE-β-CD (mg) | Dimethyl sulfoxide (mL) | Phosphate buffer solution (mL) |
|---|---|---|---|---|
| 103 | 9.66 | 310 | 0.15 | 1.25 |

### Preparation of Compound (IX) in Example 104

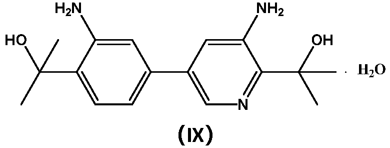

### Synthesis route:

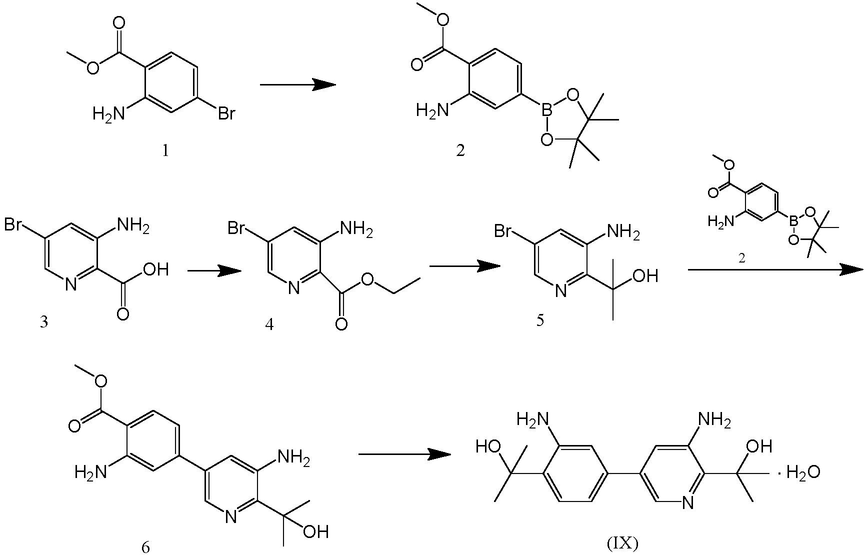

### Step 1: Preparation of Compound 2

Compound 1 (30 g, 130.4 mmol, 1 eq), bis (pinacolato) diboron (66.23 g, 260.80 mmol, 2 eq), [1,1-bis (diphenylphosphine) ferrocene] palladium (II) chloride dichloromethane adduct (5.32 g, 6.52 mmol, 0.1 eq), and potassium acetate (25.60 g, 260.80 mmol, 2 eq) were added to toluene (500 mL), and nitrogen was replaced three times. The reaction solution was stirred at 110°C for 15 hours. After the reaction was complete, the reaction solution was filtered through diatomaceous earth pad, the filtrate was concentrated, and the residue was purified by column chromatography (petroleum ether: ethyl acetate=0 to 100:6) to obtain compound 2. 1H NMR (400 MHz, CDCl3) δ 7.84 (d, J=8.0 Hz, 1H), 7.06 (s, 1H), 7.04 (d, J=8.0 Hz, 1H), 5.65 (brs, 2H), 3.87 (s, 3H), 1.35 (s, 12H).

### Step 2: Preparation of Compound 4

After dissolving compound 3 (100 g, 460.79 mmol, 1 eq) in anhydrous ethanol (1 L), concentrated sulfuric acid (225.97 g, 2.30 mol, 122.81 mL, 5 eq) and anhydrous sodium sulfate Na₂SO₄ (65.45 g, 460.79 mmol, 46.75 mL, 1 eq) were added. The reaction solution was stirred at 85°C for reaction for 48 hours. After the reaction was complete, the reaction solution was cooled to room temperature. Saturated solution of sodium bicarbonate (1L) was added dropwise to the reaction solution, a large amount of solid was generated, filtered, and the filter cake was washed with water (500 mL). The obtained solid was vacuum dried to obtain compound 4. 1H NMR (400 MHz, CDCl3) δ 8.10 (d, J=1.8 Hz, 1H), 7.26 (s, 1H), 4.47 (q, J=7.1 Hz, 2H), 1.46 (t, J=7.2 Hz, 3H).

### Step 3: Preparation of compound 5

Compound 4 (70.00 g, 285.63 mmol, 1 eq) was dissolved in tetrahydrofuran (1 L) under nitrogen protection and cooled to -78°C. Methyl lithium (1.6 M, 892.59 mL, 5 eq) was slowly added dropwise to the reaction solution, which was stirred at -78°C for 3 hours. After the reaction was completed, water (100 mL) was slowly added dropwise to quench the reaction. The solution was raised to room temperature, diluted with saturated ammonium chloride aqueous solution (500 mL), and extracted with ethyl acetate (500 mL *3). The organic phases were combined, dried with anhydrous sodium sulfate, and concentrated under reduced pressure to obtain the crude product. The crude product was beaten with n-heptane (500 mL), filtered, and dried to obtain compound 5. 1H NMR (400 MHz, CDCl3) δ 7.86 (d, J=1.9 Hz, 1H), 6.98 (d, J=1.9 Hz, 1H), 4.57 (br s, 2H), 1.57 (s, 6H).

### Step 4: Preparation of Compound 6

Compound 5 (10 g, 43.27 mmol, 1 eq), compound 2 (23.98 g, 86.55 mmol, 2 eq), [1,1-bis (diphenylphosphine) ferrocene] palladium (II) chloride dichloromethane adduct (1.77 g, 2.16 mmol, 0.05 eq), cesium carbonate (28.20 g, 86.55 mmol, 2 eq) were added to dioxane (300 mL) and water (75 mL). Nitrogen was replaced three times, and the reaction solution was stirred at 80°C for 5 hours. After the reaction was completed, the reaction solution was concentrated, and the residue was subjected to column chromatography (petroleum ether: tetrahydrofuran=0 to 100:40) to obtain crude compound 6. This crude product was heated with tetrahydrofuran (4 mL/g) to 80°C, cooled for re-crystallization, stirred at 25°C for 15 hours, and filtered. The filter cake was dried to obtain compound 6. 1H NMR (400 MHz, DMSO-d6) δ 7.94 (d, J=2.0 Hz, 1H), 7.77 (d, J=8.0 Hz, 1H), 7.17 (d, J=2.0 Hz, 1H), 6.99 (d, J=1.6 Hz, 1H), 7.77-7.75 (m, 3H), 5.69 (s, 2H), 5.50 (s, 1H), 3.81 (s, 3H), 1.52 (s, 6H).

### Step 5: Preparation of compound of formula (IX)

Compound 6 (8.78 g, 29.14 mmol, 1 eq) was dissolved in tetrahydrofuran (80 mL) and cooled to 0°C under nitrogen protection. Methyl magnesium bromide (3 M, 97.12 mL, 10 eq) was added dropwise to the reaction solution, which was stirred and reacted at 0°C for 1 hour. After the reaction was complete, saturated ammonium chloride aqueous solution (400 mL) was slowly added to quench the reaction, and the solution was extracted with ethyl acetate (400 mL *2). The organic phase was concentrated under reduced pressure, and the residue was purified with dichloromethane (3 mL/g) at 25°C, filtered and dried to obtain the product with a molecular weight of 301.40. 1H NMR (400 MHz, DMSO-d6) δ 7.88 (d, J=1.8 Hz, 1H), 7.15-7.01 (m, 2H), 6.82 (d, J=1.6 Hz, 1H), 6.69 (dd, J=1.5, 8.0 Hz, 1H), 5.59 (br s, 2H), 5.51 (br s, 2H), 5.44 (s, 1H), 5.23 (s, 1H), 1.51 (d, J=3.6 Hz, 12H). 11.9 g of the above product was weighed and added to a round-bottomed flask, added with 150 mL of methyl tert-butyl ether, stirred at 50°C for 12 hours, then cooled down to 25°C, stirred for 4 hours, filtered and dried to obtain the solid compound of formula (IX). Approximately 50 mg of the above compound of formula (IX) was further weighed and added to a 2.0 mL glass vial, added an appropriate amount of solvent or solvent mixture to form a suspension, added with a magneton, stirred on a magnetic heating stirrer (25°C/50°C) for one week, and centrifuged to obtain solid sample, which was dried in a 40°C vacuum drying oven overnight to obtain the compound of formula (IX). It has a molecular formula C₁₇H₂₃N₃O₂·H₂O, and a molecular weight of 319.40.

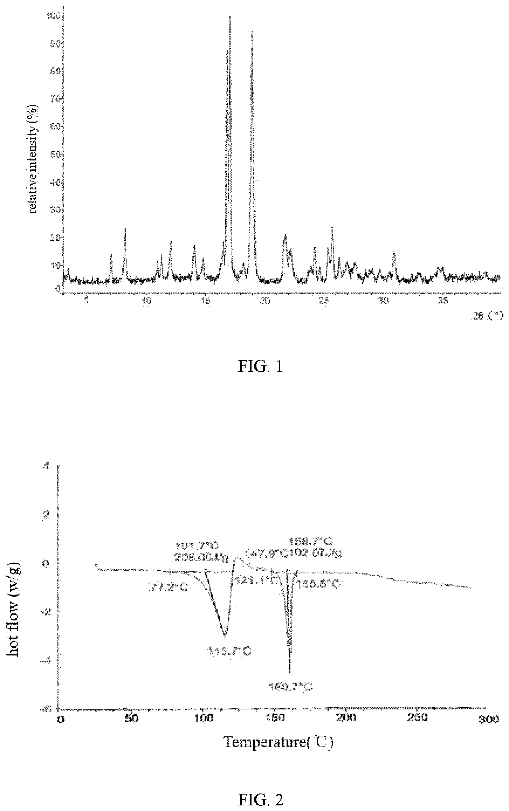

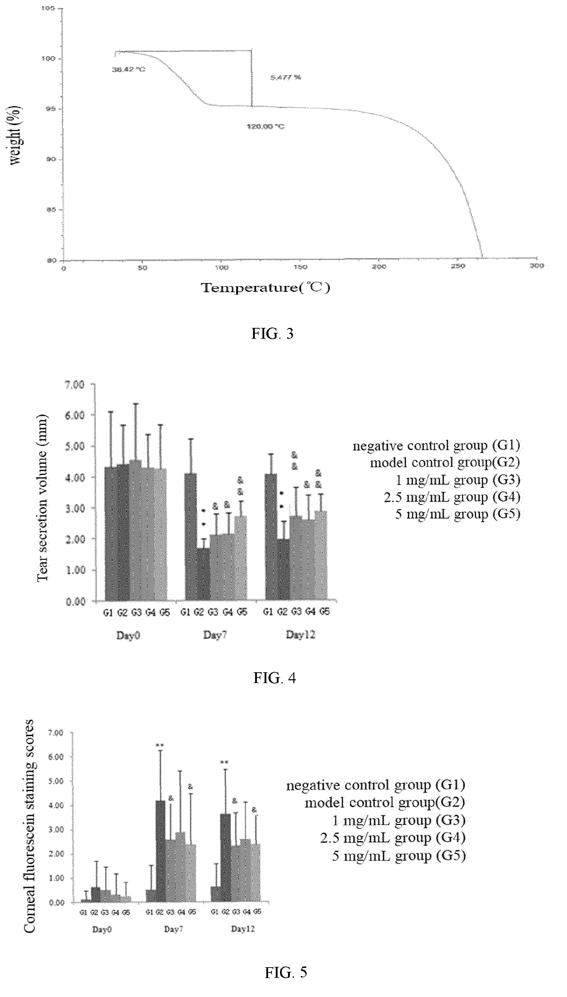

The XRPD spectrum analysis data of the above compound of formula (IX) are shown in Table 37, and the spectrum is shown in FIG. 1. The differential scanning calorimetry curves of compound (IX) show a starting point of an endothermic peak at 101.7 ± 3.0°C and 158.7 ± 3.0°C, respectively, as shown in FIG. 2. The thermogravimetric analysis curve shows a weight loss of 5.477% at 120.00°C± 3.0°C, as shown in FIG. 3.

**Table 37 XRPD spectrum analysis data of compound (IX)**

| No | 20 angle (°) | Interplanar spacing (Å) | Relative intensity (%) | No. | 20 angle (°) | Interplanar spacing (Å) | Relative intensity (%) |
|---|---|---|---|---|---|---|---|
| 1 | 7.083 | 12.4700 | 10.0 | 14 | 24.242 | 3.6683 | 13.1 |
| 2 | 8.231 | 10.7336 | 20.2 | 15 | 24.653 | 3.6081 | 5.4 |
| 3 | 11.294 | 7.8279 | 9.7 | 16 | 25.350 | 3.5105 | 12.1 |
| 4 | 12.068 | 7.3278 | 14.8 | 17 | 25.678 | 3.4664 | 19.5 |
| 5 | 14.091 | 6.2798 | 12.9 | 18 | 26.270 | 3.3896 | 7.3 |
| 6 | 14.806 | 5.9784 | 8.4 | 19 | 27.001 | 3.2995 | 6.5 |
| 7 | 16.505 | 5.3665 | 13.9 | 20 | 27.658 | 3.2226 | 5.4 |
| 8 | 17.058 | 5.1937 | 100.0 | 21 | 29.052 | 3.0710 | 4.3 |
| 9 | 18.202 | 4.8697 | 5.9 | 22 | 29.721 | 3.0034 | 4.3 |
| 10 | 18.955 | 4.6780 | 95.1 | 23 | 30.869 | 2.8943 | 10.9 |
| 11 | 21.712 | 4.0898 | 18.1 | 24 | 34.644 | 2.5871 | 4.8 |
| 12 | 22.182 | 4.0041 | 13.1 | 25 | 35.009 | 2.5609 | 5.1 |
| 13 | 23.903 | 3.7197 | 5.7 | | | | |

### Examples 105~107

According to the prescription ratio in Table 38, Examples 105 to 107 were prepared.

### Production process:

Concentrated solution preparation: adding the prescribed amount of injection water into a beaker, heating the beaker on a CNC heating type magnetic stirrer to a water temperature of 77°C± 2°C, and adding HP-β-CD in small amounts by multi-batches under stirring.

After HP-β-CD was completely dissolved, stirring was kept, and the prescribed amount of active compound was added for one time. The solution temperature was controlled at 77°C± 2°C and stirring was performed until the solution was completely clear.

Diluting concentrated solution to volume: weighing 7.5 kg of injection water at 20°C~25°C and adding into a stainless steel bucket, starting the mixer to stir, adding the concentrated solution into the stainless steel bucket, rinsing the beaker containing the concentrated solution for 4-6 times with a small amount of injection water at 20°C~25°C, transferring all the rinsing solution to the stainless steel bucket, stirring evenly, adding the prescribed amount of sodium chloride, monobasic sodium phosphate monohydrate, and dibasic sodium phosphate in sequence, stirring for dissolving, diluting the solution to 15 L with injection water at 20°C~25°C, and stirring evenly.

Filtering and filling: before production begins, conducting the integrity test of the filter cartridge, flushing and conducting steam-in-place on the mixing tank, the Blow-Fill-Seal (BFS) machine, and their material conveying pipelines, performing pure steam sterilization at a temperature of ≥ 121°C for 30 minutes, commissioning the equipment, starting filtering and filling, and conducting integrity test on the filter cartridge after production was completed.

Punching: conducting leakage detection on all punched products, then inspecting each bottle and removing any non-conforming products.

Packaging: conducting light inspection on all qualified punched products to eliminate non-conforming products, performing pillow packaging on punched products that pass leakage detection and light inspection, and testing the heat sealing effect of all packaged samples by using a vacuum constant temperature drying oven.

The results show that the prescription ratios and preparation processes of Examples 105 to 107 can produce products with controllable quality and stable properties.

**Table 38**

| Step | Material Name | Example 105 | | Example 106 | | Example 107 | |
|---|---|---|---|---|---|---|---|
| | | % | g | % | g | % | g |
| Preparat ion of concentr ated solution | Compound of formula (IX) (anhydrous) | 0.10 | 15.0 | 0.25 | 37.5 | 0.50 | 75.0 |
| | HP-β-CD | 0.70 | 105.0 | 1.75 | 262.5 | 3.50 | 525.0 |
| | Injection water | / | 105.0 | / | 262.5 | / | 525.0 |
| Diluting of concentr ated solution | Dibasic sodium phosphate | 0.81 | 121.5 | 0.81 | 121.5 | 0.81 | 121.5 |
| | Monobasic sodium phosphate monohydrate | 0.12 | 18.0 | 0.12 | 18.0 | 0.12 | 18.0 |
| | Sodium chloride | 0.40 | 60.0 | 0.36 | 54.0 | 0.27 | 40.5 |
| | Injection water | To 15 L | | To 15 L | | To 15 L | |

### Experimental Example 1: Experimental study on the effects of mouse dry eye disease model

### Experimental objective:

The therapeutic effects of Examples 105, 106, and 107 on the C57BL/6 mouse model of dry eye disease were investigated by subcutaneous injection of scopolamine hydrobromide solution into the lower limbs.

### Experimental process:

According to tear secretion volume, animals were randomly and evenly divided into five groups: negative control group (physiological saline, G1), model control group (solvent, Example 102, G2), low concentration group (1 mg/mL) (Example 105, G3), medium concentration group (2.5 mg/mL) (Example 106, G4), and high concentration group (5 mg/mL) (Example 107, G5). Each group consists of 8 animals, all female.

Animals in individual groups were injected subcutaneously with 5 mg/mL of scopolamine hydrobromide solution alternately in both lower limbs of D1, 4 times/day, 0.1 mL/time, with an interval of approximately 3 hours, for 12 consecutive days (administered 3 times on D12). The animals of the negative control group were injected subcutaneously with an equal volume of physiological saline in both lower limbs, avoiding multiple injections at the same injection point.

Animals in each of the testing groups were dosed with 3 µL/administration in both eyes on D1, 4 times/day, with an intervals of approximately 3 hours, for a total of 12 days (D12 was dosed 3 times). Animals of the negative control group and the model control group were given equal volumes of solvent by eye drops in both eyes. The tear secretion volume in both eyes was measured approximately 30 minutes after the second administration on D7 and D12, and corneal fluorescein staining scoring was performed approximately 30 minutes after the third administration on D7 and D12. After the completion of D12 index measurement, the animals were euthanized using cervical dislocation method.

### Experimental results:

The low concentration group (1 mg/mL), the medium concentration group (2.5 mg/mL), and the high concentration group (5 mg/mL) all have good therapeutic effects on the mouse dry eye disease model induced by scopolamine hydrobromide solution, mainly improving the tear secretion volume and corneal damage of the dry eye disease model mice. Based on the comprehensive evaluation of tear secretion volume (Table 39, FIG. 4) and corneal fluorescein staining (Table 40, FIG. 5) scores, the high concentration group (5 mg/mL) showed the best therapeutic effect.

**Table 39: Effects of tear secretion volume in dry eye disease model mice (x̅±s)**

| Group | Wet length of phenol red cotton thread (mm) | | |
|---|---|---|---|
| | D0 | D7 | D12 |
| Negative control group (G1) | 4.33±1.78 | 4.10±1.12 | 4.07±0.66 |
| Model control group (G2) | 4.41±1.29 | 1.70±0.30##** | 1.97±0.59##** |
| 1 mg/mL Group (G3) | 4.54±1.84 | 2.13±0.69##**& | 2.72±0.91##**&& |
| 2.5 mg/mL Group (G4) | 4.30±1.08 | 2.14±0.69##**& | 2.58±0.82##**& |
| 5 mg/mL Group (G5) | 4.28±1.41 | 2.73±0.48##**&& | 2.85±0.57##**&& |

| | | | |
|---|---|---|---|
| Note: Compared with D0, # P<0.05; ## P<0.01; compared with G1 group, *P<0.05; **P<0.01; and compared with the G2 group, & P<0.05; && P<0.01. | | | |

**Table 40: Effects of corneal fluorescein staining scores on dry eye disease model mice (x̅±s)**

| Group | Corneal fluorescein staining score | | |
|---|---|---|---|
| | D0 | D7 | D12 |
| Negative control group (G1) | 0.13±0.34 | 0.50±1.03 | 0.63±0.96# |
| Model control group (G2) | 0.63±1.09 | 4.19±2.07##** | 3.63±1.82##** |
| 1 mg/mL group (G3) | 0.50±0.97 | 2.56±1.50##**& | 2.31±1.35##**& |
| 2.5 mg/mL group (G4) | 0.31±0.87 | 2.88±2.53##** | 2.56±1.55##** |
| 5 mg/mL group (G5) | 0.25±0.58 | 2.38±2.09##**& | 2.38±1.20##**& |

| | | | |
|---|---|---|---|
| Note: Compared with D0, # P<0.05; ## P<0.01; compared with G1 group, *P<0.05; **P<0.01; and compared with the G2 group, & P<0.05; && P<0.01. | | | |

### Experimental conclusion:

Examples 105, 106, and 107 have good therapeutic effects on mouse dry eye disease models induced by scopolamine hydrobromide solution, mainly improving tear secretion volume and corneal damage in dry eye disease model mice.

### Experimental Example 2: Experimental study on the effects of hyperosmotic dry eye disease model in rats

### Experimental objective:

Investigating the therapeutic effects of Example 105 and Example 107 on the SD rat model of dry eye disease induced by eye drops of hypertonic sodium chloride solution.

### Experimental process:

20 qualified female SD rats were selected for adaptive observation, and binocular corneal fluorescein staining scoring and tear secretion volume measurement were performed on the animals, excluding animals with corneal problems, abnormal fluorescein staining, and significantly different binocular tear secretion volume. Animals with significantly different binocular tear secretion volume were selected for grouping. Based on the average binocular tear secretion volume, the animals were randomly and evenly divided into three groups: a model control group (Example 102, G1), a low concentration group (1 mg/mL) (Example 105, G2), and a high concentration group (5 mg/mL) (Example 107, G3). Each of the groups consists of 4 animals with 8 eyes, and the day of grouping was recorded as D0.

Animals in each of the groups were modeled on D1, and 20 µL of sodium chloride solution (osmotic pressure of 500 mOsmol/L) was aspirated using a pipette and dropped into the conjunctival sac of both eyes of the animals, 5 times a day, 20 µL/time, with an interval of about 2 hours, for 21 consecutive days. After dropping, the eyelids of the animals were passively closed for about 90 seconds.

All animals in each group were administered eye drops on D1, 10 µ L/eye/time, 4 times/day, with an interval of approximately 3 hours, for a total of 21 days. During the administration period, animals were weighed once a week. Binocular corneal fluorescein staining scoring, tear secretion volume measurement, and tear film break-up time measurement were performed on animals at D0, D14, and D21.

### Experimental results:

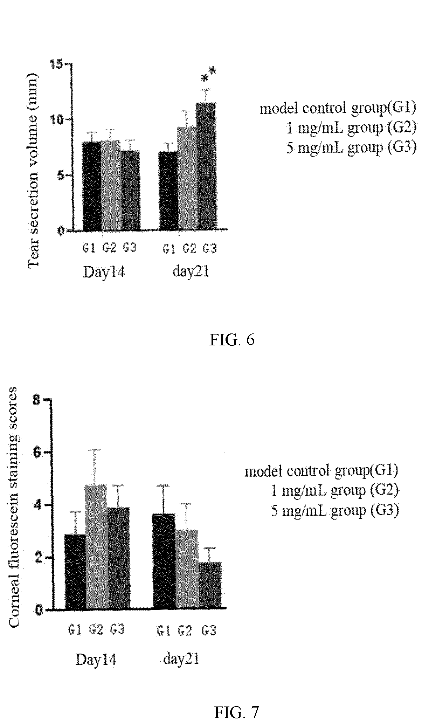

The low concentration group (1 mg/mL) and high concentration group (5 mg/mL) can increase the tear secretion volume in dry eye disease model rats, lower corneal fluorescein staining scores, and improve tear film break-up time compared to the model control group. The specific results are shown in Tables 41, 42, 43 and FIGs. 6, 7, and 8.

**Table 41 Effect of tear secretion volume in dry eye disease model rats (x̅±s)**

| Group | Wet length of phenol red cotton thread (mm) | | |
|---|---|---|---|
| | D0 | D14 | D21 |
| Model control group (G1) | 12.31±3.22 | 7.98±2.53 | 7.05±2.08 |
| 1 mg/mL (G2) | 13.78±5.25 | 8.12±2.81 | 9.24±4.07 |
| 5 mg/mL (G3) | 13.87±4.45 | 7.16±2.77 | 11.37±3.26** |

| | | | |
|---|---|---|---|
| Note: Compared with G1 group, *P<0.05; **P<0.01. | | | |

**Table 42 Effect of corneal fluorescein staining score on dry eye disease model rats x̅±s)**

| Group | Corneal fluorescein staining score | | |
|---|---|---|---|
| | D0 | D14 | D21 |
| Model control group (G1) | 0.38±0.52 | 2.88±2.47 | 3.63±2.97 |
| 1 mg/mL (G2) | 0.00±0.00 | 4.75±3.73 | 3.00±2.78 |
| 5 mg/mL (G3) | 0.13±0.35 | 3.88±2.36 | 1.75±1.49 |

| | | | |
|---|---|---|---|
| Note: Compared with G1 group, *P<0.05; **P<0.01. | | | |

**Table 43 Tear film break-up time**

| Group | Tear film break-up time (s) | |
|---|---|---|
| | D14 | D21 |
| Model control group (G1) | 7.91±2.18 | 5.15±0.73 |
| 1 mg/mL (G2) | 7.81±1.39 | 7.24±1.29** |
| 5 mg/mL (G3) | 8.71±1.30 | 7.32±1.01** |

| | | |
|---|---|---|
| Note: Compared with G1 group*P<0.05; **P<0.01 | | |

### Experimental conclusion:

The low concentration group (1 mg/mL) and high concentration group (5 mg/mL) have good therapeutic effects on the rat dry eye disease model induced by hypertonic sodium chloride solution, which was mainly manifested in that the tear secretion volume, corneal damage, and tear film stability of the dry eye disease model rats can be significantly improved after 2 weeks of administration of the drug in continuous eye drops.

### Experimental Example 3: Investigating pharmacokinetic studies in the eyes of New Zealand rabbits

Six New Zealand rabbits that passed the adaptive observation were selected and divided into two groups, with three rabbits in each group, namely the 0.5 hour sampling group and the 2 hour sampling group. Animals in each of the groups were given 5 mg/mL via eye drops in the left eye (Example 107), with a volume of 100 µL/eye for each experimental group. Plasma, corneal tissue, conjunctival tissue, and retinal tissue samples were collected from each group of animals at 0.5 and 2 hours after administration. LC-MS method was adopted to determine the concentration of active ingredients in the biological samples. The specific results are shown in Tables 44 and 45.

**Table 44 Concentration of active ingredients in individual biological samples**

| Group | Plasma drug concentration (nM) | Corneal tissue drug concentration (nmol/kg) | Conjunctival tissue drug concentration (nmol/kg) | Retinal tissue drug concentration (nmol/kg) |
|---|---|---|---|---|
| | Average | | | |
| 0.5 h | 253.7 | 36300 | 9955 | 415.1 |
| 2 h | 39.9 | 9504 | 3845 | 130.9 |

**Table 45 Concentration ratio of active ingredients in various ocular tissues and plasma**

| Group | Cornea/Plasma | Conjunctiva/Plasma | Retina/Plasma |
|---|---|---|---|
| | Drug concentration ratio | | |
| 0.5 h | 142.8 | 39.7 | 1.7 |
| 2h | 250.9 | 63.0 | 3.3 |

The experimental results showed that, after administering Example 107 to the eyes, the active ingredient had a high concentration distribution in the corneal and conjunctival tissues of the ocular surface, which is beneficial for the treatment of ocular surface diseases. The distribution concentration of active ingredients in the retinal tissue is low, which reduces the risk of adverse reactions in the fundus. Therefore, the pharmacokinetic properties of Example 107 are beneficial for the treatment of ocular surface diseases.

### Experimental Example 4: Experimental study on the effect of ragweed pollen induced allergic conjunctivitis model in mice

### Experimental objective:

A mouse model of allergic conjunctivitis was prepared by subcutaneous injection of ragweed pollen on foot pads for sensitization and local stimulation with eye drops. Clinical symptoms of mouse eyes, eye observation scores, and pathological H&E were detected, and the therapeutic effects of low concentration group (1 mg/mL) (Example 105), medium concentration group (2.5 mg/mL) (Example 106), and high concentration group (5 mg/mL) (Example 107) on allergic conjunctivitis mice were evaluated.

### Experimental process:

### 1.1 Animals

Healthy BALB/c mice, 5-7 weeks old, 50 mice, half male and half female

### 1.2 Preparation of main reagents

1.2.1 Preparation of model sensitizing drug (ragweed pollen tarsal joint injection): weighing 22.3 mg of ragweed pollen and dissolving in 7.25 mL of alum adjuvant;
1.2.2 Preparation of modeling stimulating drug (ragweed pollen eye drops): weighing 168 mg of ragweed pollen and dissolving in 1.12 mL of PBS; weighing 189 mg of ragweed pollen and dissolving in 1.26 mL of PBS (phosphate buffered saline); and weighing 144 mg of ragweed pollen and dissolving in 0.96 mL of PBS
1.2.3 Test articles

Low concentration group (1 mg/mL) (Example 105), medium concentration group (2.5 mg/mL) (Example 106), high concentration group (5 mg/mL) (Example 107), and blank group (Example 102).

### 1.3 Animal grouping, modeling, and model validation

(1) Blank group (Example 102) (solvent treatment: 4 times/animal/day, single dose: 20 µL, eye drop administration for 4 consecutive days) (N=10)
(2) Allergic conjunctivitis model group (also known as Model Group in this experiment) (solvent treatment, 4 times/animal/day, single dose: 20 µL, eye drop administration for 4 consecutive days) (N=10)
(3) Low concentration group (1 mg/mL) group (low concentration group (1 mg/mL) treatment, 4 times/animal/day, single dose: 20 µL, eye drop administration for 4 consecutive days) (N=10)
(4) Medium concentration group (2.5 mg/mL) group (medium concentration group (2.5 mg/mL) treatment, 4 times/animal/day, single dose: 20 µL, eye drop administration for 4 consecutive days) (N=10)
(5) High concentration group (5 mg/mL) group (high concentration group (5 mg/mL) treatment, 4 times/animal/day, single dose: 20 µL, eye drop administration for 4 consecutive days) (N=10)

Modeling: On day 0, ragweed pollen tarsal joint sensitization injection was injected subcutaneously into the foot pads of mice at a dose of 65 µL per mouse; and on days 10-13, ragweed pollen eye drops were applied to the right eyes of the Model Group mice for stimulation, at a dose of 10 µL/mouse/time, once a day for 4 consecutive days.

### 1.4 Animal administration

Drug intervention therapy was initiated 30 minutes after each eye drop stimulation, 4 times a day for 4 consecutive days. The blank group and Model Group were treated with equal amounts of solvent. The experimental drug treatment group was given the corresponding dose of compound treatment. The administration was divided into two doses of 20 µL per animal, 10 µL per dose, with an interval of 1 minute, 4 times per animal per day, for 4 consecutive days.

### 1.5 Testing Indicators

### 1.5.1 Clinical symptom assessment

Within 30 minutes after the last stimulation, the clinical symptoms of the mouse's eyes were observed under a microscope. Eye observation score was obtained: observing allergic reactions in the eyes, including chemosis and conjunctival hyperemia, and score them 0-3 points based on the severity (including none, mild, moderate, and severe). Scoring criteria for chemosis: 1 point for mild localized chemosis; 2 points for diffuse edema and involvement of the fornix; and 3 points for shallow narrowing of the conjunctival sac due to chemosis. Conjunctival hyperemia scoring criteria: 1 point for mild diffuse vascular hyperemia; 2 points for diffuse hyperemia and obvious near fornix; and 3 points for hyperemia with subconjunctival bleeding.

### 1.5.2 H&E staining

Experimental procedure: the tissue of the animal's right eye and eyelid were cut. First, the sample was fixed in 4% paraformaldehyde at room temperature for 4 hours. The tissue was rinsed with running water for several hours, dehydrated with 70%, 80%, and 90% ethanol solutions, and then treated with a solution of pure alcohol and xylene in equal amounts for 15 minutes. Then, the sample was permeabilized twice with xylene solution, each time for 15 minutes, until the sample was transparent, added with a mixture of half xylene and half paraffin for 15 minutes, and then added with paraffin I and paraffin II with 60 minutes of wax infiltration for each. After embedding in paraffin, the sample was sliced according to the pre-selected cross-sectional direction, baked, deparaffinized, and hydrated. The hydrated slices was stained in hematoxylin aqueous solution for 3 minutes, differentiated with hydrochloric acid ethanol differentiation solution for 15 seconds, washed slightly with water, subjected treatment by blueing solution for 15 seconds, rinsed with running water, stained with eosin for 3 minutes, rinsed with running water, dehydrated, cleared, mounted, and examined under microscope.

### 1.6 Statistical analysis

The experimental data were analyzed using GraphPad Prism 5 and IBM SPSS Statistics 19.0 software for each group using one-way ANOVA (*p<0.05, **p<0.01).

### Experimental results:

### 2.1 The effect of drugs on clinical symptoms of mouse eyes

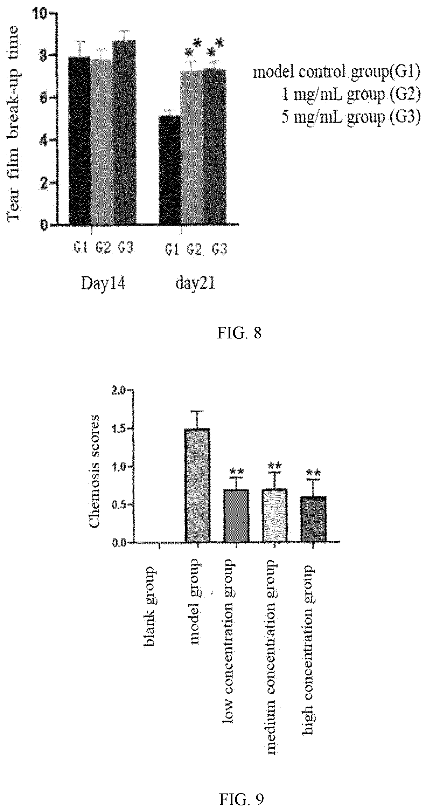

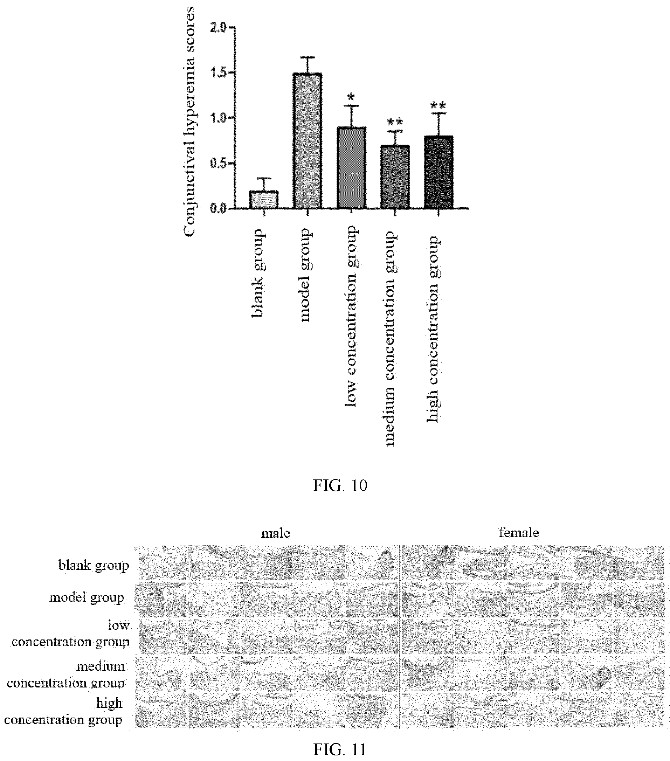

Compared with the blank group, the Model Group showed a significant increase in scores in chemosis and conjunctival hyperemia; compared with the Model Group, the low concentration group (1 mg/mL), the medium concentration group (2.5 mg/mL), and the high concentration group (5 mg/mL) significantly reduced scores (p<0.05 or p<0.01) and improved chemosis and conjunctival hyperemia. The above results suggest that the low, medium, and high concentration groups have the effect of improving chemosis and conjunctival hyperemia in mice with allergic conjunctivitis induced by ragweed pollen. The specific experimental results are shown in Figures 9 and 10.

**Table 45: Clinical symptom scores of allergic conjunctivitis mice in low concentration group, medium concentration group, and high concentration group (Mean ± SEM)**

| Group | Chemosis score | Conjunctival hyperemia score |
|---|---|---|
| Blank group | 0.0±0.00000 | 0.1±0.31623 |
| Model group | 1.5±0.70711 | 1.5±0.52705 |
| Low concentration group (1 mg/mL) | 0.7±0.48305 | 0.9±0.73786 |
| Medium concentration group (2.5 mg/mL) | 0.7±0.67495 | 0.7±0.48305 |
| High concentration group (5 mg/mL) | 0.6±0.69921 | 0.8±0.78881 |

| | | |
|---|---|---|
| Note: Compared with the model group, *p<0.05, **p<0.01. | | |

### 2.2 Effects of drugs on histopathology of allergic conjunctivitis mice

Pathological results showed that the conjunctiva of the blank group was intact and no obvious damage was observed. Individual samples showed vascular congestion, accompanied by a small amount of inflammatory cell infiltration. The Model Group showed varying degrees of thickening or thinning of the conjunctiva, disordered arrangement of conjunctival epithelial cells, significant infiltration of inflammatory cells, and no obvious capillary proliferation or hyperemia under the conjunctiva. The conjunctival structure of the low concentration (1 mg/mL) group was relatively intact, with a small amount of inflammatory cell infiltration and hyperemia visible in some samples. In the medium concentration (2.5 mg/mL) group, shedding of conjunctival epithelial cells and thickening of the conjunctiva were observed, and there were still a small number of inflammatory cell infiltration and hyperemia samples. In the high concentration (5 mg/mL) group, some samples showed irregular arrangement of conjunctival epithelial cells, thinning phenomenon, and a small number of inflammatory cell infiltration and hyperemia samples were observed. The above results suggest that low, medium, and high concentration groups can improve conjunctival structural abnormalities and inflammatory cell infiltration in mice with allergic conjunctivitis. The experimental results are shown in FIG. 11.

Experimental conclusion: the low, medium, and high concentration groups can have a good therapeutic effect on the allergic conjunctivitis model induced by ragweed pollen in mice. They mainly improve chemosis and conjunctival hyperemia in allergic conjunctivitis mice, reduce conjunctival structural abnormalities and inflammatory cell infiltration.

## Claims

1. An ophthalmic formulation comprising pyridine phenyl compound, **characterized in that**, the ophthalmic formulation comprises an active component pyridine phenyl compound and an excipient, wherein,
the excipient comprises solubilizing agent, pH modifier, osmotic pressure regulator or a combination of two or more thereof,
the active ingredient pyridine phenyl compound comprises a compound of formula (II), isomers thereof, or a pharmaceutically acceptable salt thereof, 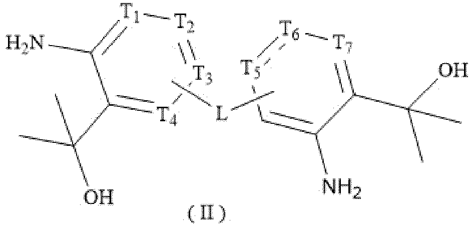 wherein,
" 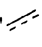 " is selected from a single bond or a double bond;
T₁, T₂, T₃, and T₄ are independently selected from N, C, or CR₁, respectively;
T₅ is selected from C, CR₅, or C=O;
T₆ is selected from C, CR₆, or N;
T₇ is selected from N or CR₇;
when T₅ is selected from C=O and T₆ is selected from N, " 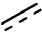 " is selected from a single bond;
L is selected from a single bond, -O-, -S-, -NR₂- or -(CR₃R₄) n-;
R₁ is selected from H, F, Cl, Br, I, OH, or NH₂;
R₂ is selected from H and a C₁₋₃ alkyl group optionally substituted with 1, 2, or 3 Rₐ;
R₃ and R₄ are independently selected from H, F, Cl, Br, I, OH, NH₂, CN, or a C₁₋₃ alkyl group optionally substituted with 1, 2, or 3 R_{b}, respectively;
R₅, R₆, and R₇ are independently selected from H, F, Cl, Br, or I, respectively;
n is selected from 1, 2, or 3; and
Rₐ and R_{b} are independently selected from H, F, Cl, Br, I, OH, NH₂, CN, or CH₃, respectively.

2. The ophthalmic formulation according to claim 1, **characterized in that**, in the compound of formula (II), isomers thereof, or a pharmaceutically acceptable salt thereof, R₂ is selected from H, CH₃, or CH₂CH₃, wherein CH₃ and CH₂CH₃ are optionally substituted with 1, 2, or 3 Rₐ.

3. The ophthalmic formulation according to claim 2, **characterized in that**, in the compound of formula (II), isomers thereof, or a pharmaceutically acceptable salt thereof, R₂ is selected from H, CH₃, or CH₂CH₃.

4. The ophthalmic formulation according to any one of claims 1-3, **characterized in that**, in the compound of formula (II), isomers thereof, or a pharmaceutically acceptable salt thereof, R₃ and R₄ are independently selected from H, F, Cl, Br, I, OH, NH₂, CN, CH₃ or CH₂CH₃, respectively, and said CH₃ and CH₂CH₃ are optionally substituted with 1, 2, or 3 R_{b}.

5. The ophthalmic formulation according to claim 4, **characterized in that**, in the compound of formula (II), isomers thereof, or a pharmaceutically acceptable salt thereof, R₃ and R₄ are independently selected from H, F, Cl, Br, I, OH, NH₂, CN, CH₃, or CH₂CH₃, respectively.

6. The ophthalmic formulation according to claim 5, **characterized in that**, in the compound of formula (II), isomers thereof, or a pharmaceutically acceptable salt thereof, L is selected from single bond, -O-, -S-, -NH-, -(CH₂)₂-, or -CH₂-.

7. The ophthalmic formulation according to any one of claims 1-6, **characterized in that**, the compound of formula (II), isomers thereof, or a pharmaceutically acceptable salt thereof is selected from: 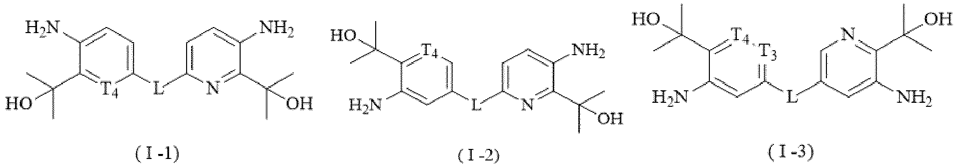 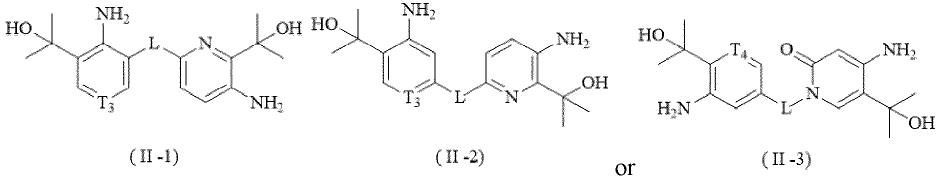 wherein,
T₃ and T₄ are independently selected from N or CR₁, respectively;
R₁ and L are as defined in any one of claims 1-6.

8. The ophthalmic formulation according to claim 7, **characterized in that**, the compound of formula (II), isomers thereof, or a pharmaceutically acceptable salt thereof is selected from: 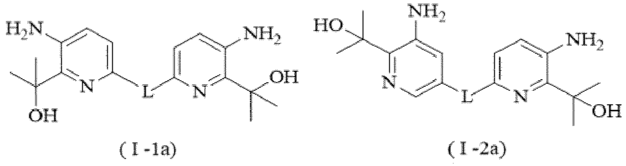 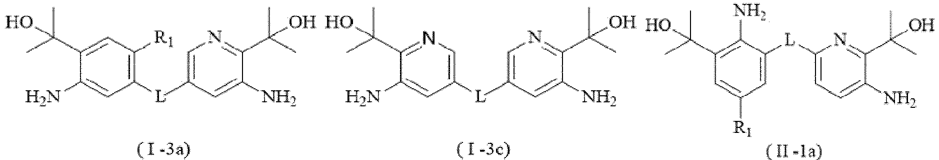 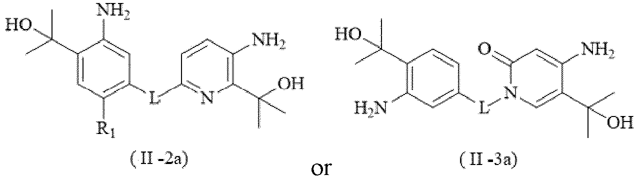 wherein,
R₁ and L are as defined in claim 7.

9. The ophthalmic formulation according to claim 8, **characterized in that**, the compound of formula (II), isomers thereof, or a pharmaceutically acceptable salt thereof is selected from:

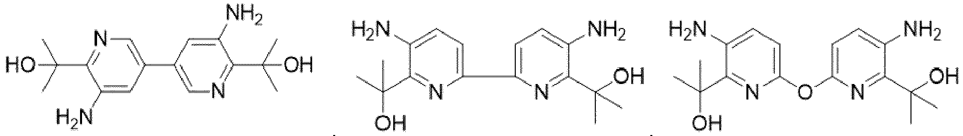

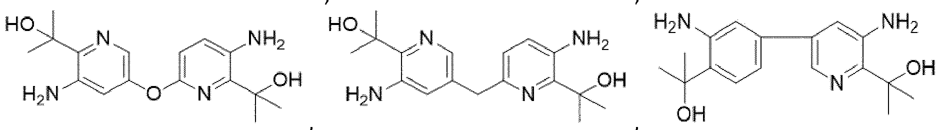

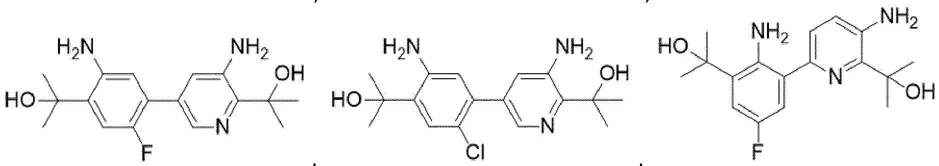

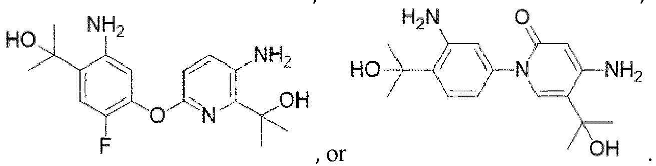

10. The ophthalmic formulation according to claim 1, **characterized in that**, the excipient further comprises antimicrobial preservative, antioxidant, freeze-drying solvent, or a combination of two or more of them.

11. The ophthalmic formulation according to claim 10, **characterized in that**, the antioxidant is selected from butylated hydroxyanisole, or vitamin E, or a combination thereof.

12. The ophthalmic formulation according to claim 10, **characterized in that**, the antimicrobial preservative is selected from benzalkonium chloride, chlorhexidine acetate, phenylmercuric acetate, or a combination of two or more of them.

13. The ophthalmic formulation according to any one of claims 1-12, **characterized in that**, the solubilizing agent is selected from methylated-β-cyclodextrin, hydroxypropyl betadex, hydroxypropyl-γ-cyclodextrin, sulfobutylether-β-cyclodextrin, poloxamer 407, Polysorbate 80, povidone, polyethylene glycol, propylene glycol, glycerol, or a combination of two or more of them.

14. The ophthalmic formulation according to any one of claims 1-12, **characterized in that**, the pH modifier is selected from monobasic sodium phosphate monohydrate, dibasic sodium phosphate, borax, boric acid, citric acid dihydrate, hydrochloric acid, sodium hydroxide, or a combination of two or more of them.

15. The ophthalmic formulation according to any one of claims 1-12, **characterized in that**, the osmotic pressure regulator is selected from sodium chloride, boric acid, borax, glucose, mannitol, or a combination of two or more of them.

16. The ophthalmic formulation according to any one of claims 1-15, **characterized in that**, the ophthalmic formulation comprises a pyridine phenyl compound, one or more solubilizing agents, one or more pH modifiers, one or more osmotic pressure regulators, one or more antimicrobial preservatives, and one or more antioxidants, wherein the pyridine phenyl compound comprises the compound of formula (III), formula (IV), formula (V), formula (VI), formula (VII), or formula (VIII), its isomers or pharmaceutically acceptable salts:

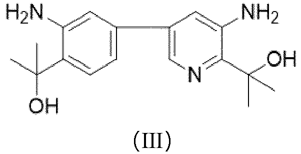

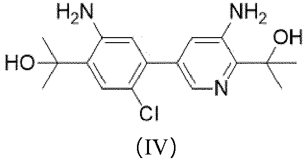

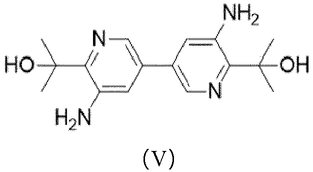

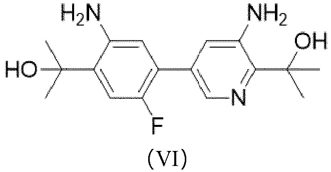

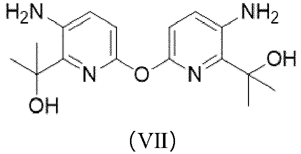

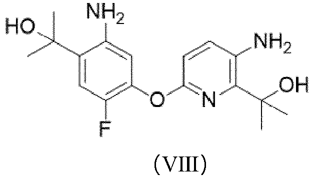

17. The ophthalmic formulation according to claim 16, **characterized in that**, the compound of formula (III) further comprises a monohydrate of formula (III) (compound of formula IX), its isomer or its pharmaceutically acceptable salt:

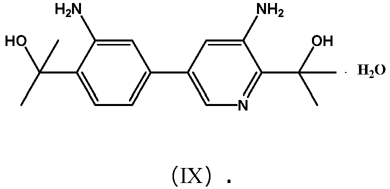

18. The ophthalmic formulation according to any one of claims 1-17, **characterized in that**, the content of the active ingredient is 0.05-0.6% w/v, preferably 0.1% w/v, 0.11% w/v, 0.12% w/v, 0.13% w/v, 0.14% w/v, 0.15% w/v, 0.16% w/v, 0.17% w/v, 0.18% w/v, 0.19% w/v, 0.2% w/v, 0.21% w/v, 0.22% w/v, 0.23% w/v, 0.24% w/v, 0.25% w/v, 0.26% w/v, 0.27% w/v, 0.28% w/v, 0.29% w/v, 0.3% w/v, 0.31% w/v, 0.32% w/v, 0.33% w/v 0.34% w/v, 0.35% w/v, 0.36% w/v, 0.37% w/v, 0.38% w/v, 0.39% w/v, 0.4% w/v, 0.41% w/v, 0.42% w/v, 0.43% w/v, 0.44% w/v, 0.45% w/v, 0.46% w/v, 0.47% w/v, 0.48% w/v, 0.49% w/v, 0.5% w/v, 0.51% w/v, 0.52% w/v, 0.53% w/v, 0.54% w/v, or 0.55% w/v.

19. The ophthalmic formulation according to claim 17, **characterized in that**, the solubilizing agent is selected from methylated-β-cyclodextrin, hydroxypropyl betadex , hydroxypropyl-γ-cyclodextrin , sulfobutylether-β-cyclodextrin, poloxamer 407, Polysorbate 80, povidone , polyethylene glycol , propylene glycol, glycerol, or a combination of two or more of them.

20. The ophthalmic formulation according to claim 19, **characterized in that**, the content of the solubilizing agent is 0.2% to 15% w/v, preferably 0.3% w/v, 0.4% w/v, 0.5% w/v, 0.6% w/v, 0.65% w/v, 0.7% w/v, 0.75% w/v, 0.8% w/v, 0.85% w/v, 0.9% w/v, 0.95% w/v, 1.0% w/v, 1.1% w/v, 1.2% w/v, 1.3% w/v, 1.4% w/v, 1.5% w/v, 1.55% w/v, 1.6% w/v, 1.65% w/v, 1.7% w/v, 1.75% w/v, 1.8% w/v, 1.9% w/v, 2.0% w/v 2.1% w/v, 2.2% w/v, 2.3% w/v, 2.4% w/v, 2.5% w/v, 2.6% w/v, 2.7% w/v, 2.8% w/v, 2.9% w/v, 3.0% w/v, 3.3% w/v, 3.5% w/v, 3.6% w/v, 3.8% w/v, 4.0% w/v, 4.2% w/v, 4.4% w/v, 4.5% w/v, 4.6% w/v, 4.8% w/v, 5.0% w/v, 5.2% w/v, 5.4% w/v, 5.5% w/v, 5.6% w/v, 5.8% w/v, 6.0% w/v, 6.2% w/v, 6.5% w/v, 6.8% w/v 7.0% w/v, 7.2% w/v, 7.4% w/v, 7.5% w/v, 7.6% w/v, 7.8% w/v, 8.0% w/v, 8.2% w/v, 8.5% w/v, 8.6% w/v 8.8% w/v, 9.0% w/v, 9.2% w/v, 9.4% w/v, 9.5% w/v, 9.6% w/v, 9.8% w/v, or 9.9% w/v.

21. The ophthalmic formulation according to claim 19, **characterized in that**, the content of hydroxypropyl betadex is 0.5% w/v~12% w/v, preferably 0.7% w/v~10% w/v, more preferably 1% w/v~8% w/v, most preferably 1.2% w/v, 1.7% w/v, 1.75% w/v, 2% w/v, 2.5% w/v, 2.8% w/v, 3% w/v, 3.3% w/v, 3.5% w/v, 4% w/v, 4.4% w/v, 5% w/v, 5.5% w/v, 6% w/v, 7% w/v, 7.5% w/v, or 8% w/v.

22. The ophthalmic formulation according to claim 18, **characterized in that**, the content of sulfobutylether-β-cyclodextrin is 4% to 13%, preferably 4.3% w/v, 5% w/v, 6% w/v, 7% w/v, 8% w/v, 9% w/v, 10% w/v, 11% w/v, 12% w/v, or 12.5% w/v.

23. The ophthalmic formulation according to claim 17, **characterized in that**, the content of the pH modifier is 0.12-20% w/v, preferably 0.2% w/v, 0.25% w/v, 0.3% w/v, 0.35% w/v, 0.4% w/v, 0.47% w/v, 0.5% w/v, 0.6% w/v, 0.7% w/v, 0.8% w/v, 0.81% w/v, 0.9% w/v, 1% w/v, 2% w/v, 3% w/v, 4% w/v, 5% w/v, 6% w/v, 7% w/v, 8% w/v, 9% w/v, 10% w/v, 11% w/v, 12% w/v, 13% w/v, 14% w/v, 15%. w/v, 16% w/v, 17% w/v, 18% w/v, 19% w/v, or 20% w/v.

24. The ophthalmic formulation according to claim 21, **characterized in that**, the pH modifier is selected from monobasic sodium phosphate monohydrate, dibasic sodium phosphate, or a combination of two of them.

25. The ophthalmic formulation according to claim 23, **characterized in that**, the content of monobasic sodium phosphate monohydrate is 0.1% w/v~0.5% w/v, preferably 0.12% w/v~0.45% w/v, more preferably 0.15% w/v, 0.2% w/v, 0.25% w/v, 0.3% w/v, 0.35% w/v, or 0.4% w/v.

26. The ophthalmic formulation according to claim 22, **characterized in that**, the content of dibasic sodium phosphate is 0.3% w/v~1% w/v, preferably 0.4% w/v~0.9% w/v, more preferably 0.45% w/v, 0.47% w/v, 0.5% w/v, 0.55% w/v, 0.6% w/v, 0.65% w/v, 0.7% w/v, 0.75% w/v, or 0.81% w/v.

27. The ophthalmic formulation according to claim 17, **characterized in that**, the content of the osmotic pressure regulator is 0.1% to 1% w/v, preferably 0.2% w/v, 0.21% w/v, 0.22% w/v, 0.23% w/v, 0.24% w/v, 0.25% w/v, 0.26% w/v, 0.27% w/v, 0.28% w/v, 0.29% w/v, 0.3% w/v, 0.31% w/v, 0.32% w/v, 0.33% w/v, 0.34% w/v, 0.35% w/v, 0.36% w/v, 0.37% w/v, 0.38% w/v, 0.39% w/v, 0.4% w/v, 0.42% w/v, 0.44% w/v, 0.45% w/v 0.46% w/v, 0.48% w/v, 0.5% w/v, 0.55% w/v, 0.6% w/v, 0.65% w/v, 0.7% w/v, 0.75% w/v, 0.8% w/v, 0.85% w/v, 0.9% w/v, or 0.95% w/v.

28. The ophthalmic formulation according to claim 17, **characterized in that**, the osmotic pressure regulator is selected from sodium chloride.

29. The ophthalmic formulation according to claim 28, **characterized in that**, the content of sodium chloride is 0.2% w/v~0.8% w/v, preferably 0.25% w/v~0.7% w/v, more preferably 0.26% w/v, 0.27% w/v, 0.3% w/v, 0.34% w/v, 0.36% w/v, 0.4% w/v, 0.45% w/v, 0.5% w/v, 0.55% w/v, 0.6% w/v, or 0.65% w/v.

30. The ophthalmic formulation according to claim 17, **characterized in that**, the content of the antimicrobial preservative is 0.001% w/v~0.02% w/v, preferably 0.002% w/v~0.018% w/v, further preferably 0.003% w/v~0.016% w/v, most preferably 0.004% w/v, 0.0045% w/v, 0.005% w/v, 0.0055% w/v, 0.006% w/v, 0.0065% w/v, 0.007% w/v, 0.0075% w/v, 0.008% w/v, 0.0085% w/v, 0.009% w/v, 0.01% w/v, 0.012% w/v, 0.014% w/v, or 0.015% w/v.

31. The ophthalmic formulation according to claim 17, **characterized in that**, the antimicrobial preservative is selected from benzalkonium chloride or chlorhexidine acetate.

32. The ophthalmic formulation according to claim 31, **characterized in that**, the content of benzalkonium chloride is 0.001% w/v~0.02% w/v, preferably 0.002% w/v~0.018% w/v, further preferably 0.003% w/v~0.016% w/v, most preferably 0.004% w/v, 0.0045% w/v, 0.005% w/v, 0.0055% w/v, 0.006% w/v, 0.0065% w/v, 0.007% w/v, 0.0075% w/v, 0.008% w/v, 0.0085% w/v, 0.009% w/v, 0.01% w/v, 0.012% w/v, 0.014% w/v, or 0.015% w/v.

33. The ophthalmic formulation according to claim 31, **characterized in that**, the content of chlorhexidine acetate is 0.001% w/v~0.02% w/v, preferably 0.002% w/v~0.018% w/v, further preferably 0.003% w/v~0.016% w/v, most preferably 0.004% w/v, 0.0045% w/v, 0.005% w/v, 0.0055% w/v, 0.006% w/v, 0.0065% w/v, 0.007% w/v, 0.0075% w/v, 0.008% w/v, 0.0085% w/v, 0.009% w/v, 0.01% w/v, 0.012% w/v, 0.014% w/v, or 0.015% w/v.

34. The ophthalmic formulation according to claim 17, **characterized in that**, the content of the antioxidant is 0.1% ~ 0.8% w/v, preferably 0.2% w/v, 0.3% w/v, 0.4% w/v, 0.5% w/v, 0.6% w/v, or 0.7% w/v.

35. The ophthalmic formulation according to claim 17, **characterized in that**, the ophthalmic formulation comprises:
a compound of formula (IX) and its isomers or pharmaceutically acceptable salts, preferably with a content of 0.1% w/v~0.5% w/v;
hydroxypropyl betadex, preferably with a content of 0.7% w/v~3.5% w/v, preferably 1.75% w/v, 3.5% w/v, or 0.7% w/v;
dibasic sodium phosphate, preferably with a content of 0.81% w/v;
monobasic sodium phosphate monohydrate, preferably with a content of 0.12% w/v;
sodium chloride, with a content of 0.25% w/v~0.45% w/v, preferably 0.27% w/v, 0.36% w/v, 0.4% w/v; and
chlorhexidine acetate, preferably with a content of 0.01% w/v.

36. The ophthalmic formulation according to any one of claims 1-35, **characterized in that**, the pH range of the ophthalmic formulation is 5.0~9.0, preferably 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 6.91, 6.92, 6.93, 6.94, 6.95, 6.96, 6.97, 6.98, 6.99, 7, 7.1, 7.2, 7.3, 7.4, 7.5, 7.6, 7.7, 7.72, 7.73, 7.74, 7.75, 7.76, 7.77, 7.8, 7.9, 8.0, 8.1, 8.2, 8.3, 8.4, 8.5, 8.53, 8.54, 8.55 8.56, 8.57, 8.58, 8.59, 8.6, 8.7, 8.8or 8.9.

37. The ophthalmic formulation according to claim 10, **characterized in that**, the freeze-drying solvent is selected from 95% ethanol, tert-butanol, isopropanol, or acetonitrile.

38. A preparation method of the ophthalmic formulation according to any one of claims 1-37, wherein the preparation method adopts rotary evaporation process, concentrated solution-diluting method, or freeze-drying method.

39. The preparation method of the ophthalmic formulation according to any one of claims 1 -37, wherein the formulation is liquid formulation, preferably eye drop, eyewash or intraocular injection solution; ophthalmic semisolid formulation, preferably eye ointment, eye cream, or eye gel; or ophthalmic solid formulations, preferably eye film, eye pellet, and intraocular insert.

40. Use of the ophthalmic formulation according to any one of claims 1-37 for the preparation of a drug for treating ophthalmic diseases, wherein the ophthalmic diseases are preferably dry eye disease, allergic conjunctivitis, macular degeneration, cataracts, keratoconus, bullous keratopathy, Fuchs endothelial corneal dystrophy, ocular cicatricial pemphigoid, meibomian gland dysfunction, uveitis, scleritis, Stevens-Johnson syndrome, ocular rosacea, or Sjögren syndrome.
